(19) Europäisches Patentamt
European Patent Office
Office européen des brevets

(11) **EP 0 485 683 B1**

(12) **EUROPEAN PATENT SPECIFICATION**

(45) Date of publication and mention
of the grant of the patent:
**10.01.2001 Bulletin 2001/02**

(51) Int Cl.[7]: **C07C 15/073**, C01B 33/36,
C07C 2/66, B01J 29/40,
C07C 6/12

(21) Application number: **90870211.1**

(22) Date of filing: **13.11.1990**

(54) **Preparation of alkylated aromatics**

Herstellung alkylierter Aromaten

Préparation d'aromatiques alkylées

(84) Designated Contracting States:
**BE DE FR GB IT NL**

(43) Date of publication of application:
**20.05.1992 Bulletin 1992/21**

(73) Proprietor: **FINA TECHNOLOGY, INC.**
**Houston, Texas 77267-4412 (US)**

(72) Inventors:
• **Butler, James R.**
**Houston, Texas 77059 (US)**
• **Merril, James T.**
**Katy, Texas 77449 (US)**
• **Shamshoum, Edwar**
**Houston, Texas 77062 (US)**

(74) Representative: **Leyder, Francis et al**
**c/o Fina Research S.A.**
**Dept. Brevets**
**Zone Industrielle C**
**7181 Seneffe (Feluy) (BE)**

(56) References cited:
**DE-A- 3 437 615**     **US-A- 4 169 111**
**US-A- 4 459 426**     **US-A- 4 555 311**
**US-A- 4 891 458**

**Description**

This invention relates to an alkylation transalkylation process for the preparation of alkylated aromatics.

BACKGROUND OF THE INVENTION

Molecular sieves and their use in aromatic conversion processes are well known in the chemical processing and refining industry. Aromatic conversion reactions of considerable commercial importance include the alkylation of aromatic compounds such as in the production of ethyltoluene, xylene, ethylbenzene, cumene, or higher alkyl aromatics and in disproportionation reactions such as toluene disproportionation, xylene isomerization, or the transalkylation of polyalkylbenzenes to monoalkylbenzenes. Such alkylation, transalkylation or other aromatic conversion processes may be carried out in the liquid phase, in the vapor phase, or under conditions in which both liquid and vapor phases exist.

An example of vapor phase alkylation is found in U.S. Patent No. 4,107,224 to Dwyer. Here, vapor phase ethylation of benzene over a zeolite catalyst is accomplished in a down flow reactor. The output from the reactor is passed to a separation system in which ethylbenzene product is recovered, with the recycle of polyethylbenzenes to the alkylation reactor where they undergo transalkylation reactions with benzene. The Dwyer catalysts are characterized in terms of those having a constraint index within the approximate range of 1 to 12 and include, with the constraint index in parenthesis, ZSM-5 (8.3), ZSM-11 (8.7), ZSM-12 (2), ZSM-35 (4.5), ZSM-38 (2), and similar materials. Various molecular sieves, including, inter alia, zeolite beta (constraint index 0.6), are disclosed as having constraint indices outside of the range suitable for the Dwyer ethylbenzene production process.

U.S. Patent No. 3,551,510 to Pollitzer et al. discloses an alkylation-transalkylation process in which the output from the alkylation reaction zone is passed directly to the transalkylation zone. In Pollitzer, alkylation is carried out using an alkylating agent, characterized as an olefin acting compound, over a solid phosphoric acid alkylation catalyst. The olefin acting compound may be selected from materials such as monoolefins, diolefins, polyolefins, actylenic hydrocarbons, alkyl halides, alcohols, ethers and esters. The output from the alkylation reaction zone, which includes polyethylbenzenes, is supplied to a transalkylation reaction zone along with an aromatic substrate, e.g., benzene. The transalkylation zone contains an acid extracted crystalline aluminosilicate catalyst, specifically mordenite, and is operated in an upflow mode. Exemplary transalkylation conditions include a liquid hourly space velocity of 1.0, a pressure of 500 psig and a temperature of 250°C. The output from the transalkylation zone is supplied to a separation zone from which a polyalkylaromatic, e.g., polyethylbenzene, is withdrawn and recycled to the alkylation zone.

Another alkylation-transalkylation process is disclosed in U.S. Patent No. 4,008,290 to Ward. Ward, like the patent to Pollitzer, discloses the use of a solid phosphoric acid catalyst in the alkylation zone. In Ward, benzene is reacted with propylene to produce cumene. The output from the alkylation reactor is split so thata portion principally benzene and cumene, is recycled to the alkylation reactor. Another portion containing principally benzene, cumene, propane and di- and tri- isopropylbenzene is supplied to a separation zone. In the separation zone a di- and tri-isopropylbenzene rich stream is separated and supplied along with benzene to a transalkylation zone which contains a solid phosphoric acid catalyst. A cumene rich effluent from the transalkylation zone is recycled to the separation zone.

U.S. Patent No. 4,169,111 to Wight discloses an alkylation-transalkylation process for the manufacture of ethylbenzene employing crystalline aluminosilicates in the alkylation and transalkylation reactors. The catalysts in the alkylation and transalkylation reactors may be the same or different and include low sodium content zeolites, preferably less than 0.5 weight percent $Na_2O$, having silica/alumina mole ratios between 2 and 80 and preferably between 4-12. Exemplary zeolites include molecular sieves of the X, Y, L, B, ZSM-5 and omega crystal types with steam stabilized Y zeolite containing about 0.2% $Na_2O$ being preferred. The alkylation reactor is operated in a downflow mode and under temperature and pressure conditions in which some liquid phase is present. The transalkylation reactor, which is described as generally requiring higher temperatures than the optimum temperature for alkylation in order to achieve maximum transalkylation efficiency, is also operated in a downflow mode. In Wight, the output from the alkylation reactor is cooled and supplied to a benzene column from which benzene is recycled to the alkylation reactor. The bottoms fraction from the benzene column is supplied to an ethylbenzene column from which ethylbenzene is recovered as the process product. The bottoms product from the ethylbenzene column is supplied to a third column which is operated to provide a substantially pure diethylbenzene overheads fraction which contains from 10 to 90%, preferably 20 to 60% of the total diethylbenzene feed to the column. The diethylbenzene overheads fraction is recycled to the alkylation reactor while a side cut containing the remaining diethylbenzene and triethylbenzene and higher molecular weight compounds is supplied to the transalkylation reactor along with benzene. The effluent from the transalkylation reactor is recycled to the benzene column.

U.S. Patent 4,891,458 to Innes discloses liquid phase alkylation followed by liquid phase transalkylation with some optional transalkylation which may be blended with the alkylation reactor effluent. This is carried in the presence of a catalyst comprising zeolite beta.

U.S. Patent No. 4,774,377 to Barger et al. discloses an alkylation/transalkylation process which, like the above-described Wight process, involves the use of separate alkylation and transalkylation reaction zones, with recycle of the transalkylated product to an intermediate separation zone. In the Barger process, the temperature and pressure conditions are adjusted so that the alkylation and transalkylation reactions take place in essentially the liquid phase. The transalkylation catalyst is an aluminosilicate molecular sieve including X-type, Y-type, ultrastable-Y, L-type, omega type and mordenite type zeolites with the latter being preferred. The catalyst employed in the alkylation reaction zone is a solid phosphoric acid containing material. Aluminosilicate alkylation catalysts may also be employed and water varying from 0.01 to 6 volume percent is supplied to the alkylation reaction zone. The output from the alkylation reaction zone is supplied to first and second separation zones. Water is recovered in the first separation zone. In the second reaction zone intermediate aromatic products and trialkylaromatic and heavier products are separated to provide an input to the transalkylation reaction zone having only dialkyl aromatic components, or diethylbenzene in the case of an ethylbenzene manufacturing procedure or diisopropylbenzene in the case of cumene production. A benzene substrate is also supplied to the transalkylation zone for the transalkylation reaction and the output from the transalkylation zone is recycled to the first separation zone. The alkylation and transalkylation zones may be operated in downflow, upflow, or horizontal flow configurations.

As noted previously, various zeolitic molecular sieves are known for use in alkylation and/or transalkylation procedures. As indicated in the aforementioned U.S. Patent No. 4,107,224 to Dwyer, certain molecular sieves, including zeolite beta, are characterized as being unsuitable for use in the production of relatively low molecular weight alkylbenzenes such as ethylbenzene. In contrast to the relatively low molecular weight alkylbenzenes disclosed in the Dwyer et al. patent, U.S. Patent No. 4,301,316 to Young, discloses the use of molecular sieves to produce relatively high molecular weight alkylbenzenes which may be used as precursors for alkyl aryl sulfonate detergents. In Young, relatively long chain length alkylating agents having one or more reactive alkyl groups of at least 5 carbon atoms are employed in the alkylation of benzene in the presence of a crystalline zeolite alkylation catalyst. The reactants may be in the vapor phase or the liquid phase and the zeolite catalysts may be modified or unmodified. Preferred zeolite catalysts include zeolite beta, ZSM-4, ZSM-20, ZSM-38, and synthetic and naturally occurring isotypes thereof such as zeolite omega and others. As described in Young, the zeolites may be subject to various chemical treatments including alumina extraction and combination with one or more metal components such as the metals of groups IIB, III, IV, VI, VIIA, and VIII. The zeolites may also be subjected to thermal treatments including steaming or calcination in air, hydrogen or an inert gas. Specifically disclosed is the reaction of benzene and 1-dodecene over zeolite beta ($SiO_2$/$Al_2O_3$ = 175) in a flow reactor at 250°C and 600 psig.

U.S. Patent No. 4,185,040 to Ward et al. discloses an alkylation process employing a molecular sieve catalyst of low sodium content which is said to be especially useful in the production of ethylbenzene from benzene and ethylene and cumene from benzene and propylene. The $Na_2O$ content of the zeolite should be less than 0.7 weight percent and preferably less than 0.5 weight percent. Examples of suitable zeolites include molecular sieves of the X, Y, L, B, ZSM-5, and omega crystal types, with steam stabilized hydrogen Y zeolite being preferred. Specifically disclosed is a steam stabilized ammonium Y zeolite containing about 0.2% $Na_2O$. The alkylation process may be carried out with either upward or downward flow, the latter being preferred, and preferably under temperature and pressure conditions so that at least some liquid phase is present, at least until substantially all of the olefin alkylating agent is consumed. Ward et al. states that rapid catalyst deactivation occurs under most alkylating conditions when no liquid phase is present.

Another alkylation procedure is disclosed in European Patent Application No. 272,830 to Ratcliffe et al. The Ratcliffe procedure employs molecular sieve alkylation catalysts which have been treated in a manner to improve selectivity to monoalkylation, specifically in the propylation of benzene to produce cumene. Selectivity is said to be increased by at least one percentage point by first depositing a carbonaceous material on the catalyst and then subjecting the resultant carbon containing catalyst particles to combustion. Specific zeolitic crystalline molecular sieves include those selected from the group of Y zeolites, fluorided Y zeolites, X zeolites, zeolite beta, zeolite L, and zeolite omega. The zeolites may be modified to arrive at products of reduced alumina content and reduced sodium content. A preferred zeolite is Y zeolite produced by first ammonium exchanging to a sodium content of 0.6-5.0 wt.%, expressed as $Na_2O$, calcining at a temperature of 315°C-900°C in the presence of steam, and then ammonium exchanging the steam-calcined zeolite to obtain a product having less than 1.0 weight percent and preferably less than about 0.2 weight percent sodium, expressed as $Na_2O$.

Zeolite beta referred to in certain of the references addressed previously is a crystalline aluminosilicate molecular sieve zeolite which finds application in a number of industrial processes including as a catalyst in various hydrocarbon conversion reactions such as hydrocracking, hydroisomerization and dewaxing. Zeolite beta, like many other molecular sieve zeolites, is synthesized by the hydrothermal digestion of a reaction mixture comprising silica, alumina, an alkali alkaline earth metal and an organic templating agent. The organic agent acts as a template in the nucleation and growth of the zeolite beta crystals. Once the crystals are formed, it is conventional practice to carry out a calcination treatment in order to remove the organic material from the interstitial channels of the molecular sieve network.

Crystalline zeolite beta, which is identified by its x-ray diffraction pattern, and basic procedures for its preparation are disclosed in U.S. Patent No. 3,308,069 to Wadlinger et al. The chemical composition of zeolite beta in the as synthesized form as disclosed in the patent to Wadlinger et al. may be characterized as follows:

$$[X \tfrac{m}{2} (1.0\ 1\text{-}x)\ TEA]\ AlO_2 \cdot ySiO_2 \cdot WH_2O$$

wherein: X is less than 1,

.    m is at least one cation, usually an alkali metal or alkaline earth metal, more specifically sodium,
.    n is the valence of m, y is from 5 to 100,
.    W is about 4, and
.    TEA represents the tetraethylammonium ion.

As described in Wadlinger et al., zeolite beta may be formed from a mixture in water of tetraethylammonium hydroxide and suitable sources of sodium monoxide (or hydroxide), alumina, and silica. Typical reaction mixture compositions, in terms of mole ratios, fall within the following ranges:

.    $SiO_2/Al_2O_3$ - from 10 to 200;
.    $Na_2O$/tetraethylammonium hydroxide (TEAOH) - from 0-0.1;
.    $TEAOH/SiO_2$ - from 0.1-1.0;
.    $H_2O/TEAOH$ - from 20 to 75.

The resulting reaction mixture can be heated at a temperature of about 75° to about 200°C until crystallization of the molecular sieve occurs. The crystallized product can be separated from the reaction mixture by filtration or centrifuging and then washed with water and dried to remove water from the molecular sieve network. The product can then be calcined in air or in an inert atmosphere in order to remove the templating agent as described above.

The Wadlinger patent discloses that the catalytic materials can be prepared by calcining the original sodium form of the zeolite beta and/or replacing the major portion of the sodium ions with other metallic or ammoniacal ions. Specifically disclosed in Wadlinger (Example 2) is a composition containing after calcination in air at 55°C, 0.7 mole percent $Na_2O$. Disclosed in Example 8 is a product formed by treating a dried product which was exchanged continuously for 48 hours with 2% solution of ammonium chloride. After washing free of excess chlorine ions, the catalyst was dried and calcined for 3 hours at 538°C (1000°F) to produce an acid beta aluminosilicate having 0.07% Na content.

Various other procedures are known for the synthesis of zeolite beta. European Patent Application 159,846 by Rubin, discloses the synthesis of zeolite beta having a silica/alumina . mole ratio of up to 300 employing a templating agent formed by the combination of dimethylbenzylamine and benzyl halide. The hydrothermal digestion procedure in which the crystals are formed is carried out at a temperature below 175°C in order to avoid the formation of undesirable side effects. The zeolite beta produced in accordance with the Rubin application, when employed either as an absorbent or a catalyst, can be at least partially dehydrated by heating at a temperature of 200°-600°C in an air or nitrogen atmosphere for 1-48 hours. The inorganic cations of freshly synthesized zeolite beta can be decomposed by heating to a temperature up to about 550°C for 1-48 hours. Zeolite beta prepared in accordance with the Rubin process can have the original cations associated therewith replaced by a wide variety of other cations including hydrogen, ammonium and metal cations and mixtures thereof.

European Patent Application 165,208 by Bruce et al., discloses a procedure for the preparation of zeolite beta similar to that disclosed in the aforementioned Rubin application except that the templating agent is a dibenzyl dimethyl ammonium halide or hydroxide with the silica/alumina components employed to provide a silica/alumina mole ratio in the synthesized product of 20-250.

U.S. Patent 4,642,226 to Calvert et al. discloses a process for the preparation of zeolite beta which is similar to those disclosed in the aforementioned European patent applications and which employs dibenzyl dimethylammonium hydroxide or chloride as a templating agent. The reaction mixture in Calvert is heated at a temperature of 80° to 175°C for 1 to 120 days. The Calvert patent states that the zeolite beta can be used in either the organic nitrogen-containing an alkali metal containing form, the alkali metal form and hydrogen form or another univalent or multivalent cationic form. Calvert also discloses that zeolite beta can be used in intimate combination with a metallic component, e.g., a hydrogenation component such as tungsten, vanadium, molybdenum, rhenium, nickel, cobalt, chromium, manganese, or a noble metal such as platinum or palladium. The patent further states that the zeolite beta should be at least partially dehydrated when employed either as an absorbent or as a catalyst or as a hydrocarbon conversion catalyst. Chlorides, nitrates, and sulfates are disclosed as ion exchange agents. Calvert et al. discloses zeolite beta of relatively low sodium content, e.g., 0.14 wt.% Na and 0.11 wt.% Na.

Another process for the preparation of zeolite beta is disclosed in European Patent Application 164,939 by Calvert. The synthesis procedures disclosed here are similar to those in the above-mentioned references, except that a tetraethylammonium bromide or hydroxide templating agent is employed to produce a partially crystalline product of extremely high silica/alumina ratio which is said to be less expensive than fully crystalline zeolite beta which is dealuminized to provide a corresponding silica/ alumina mole ratio. The digestion period in this procedure is for a period of 1-7 days at a temperature of 90°-200°C. The silica/alumina ratio of the zeolite beta produced here ranges from 20-1000 and is preferably greater than 200.

European Application 186,447 by Kennedy et al., discloses the use of zeolite beta in catalytic cracking processes. The zeolite beta may be used in the as-synthesized form following calcination and be of either low or high silica/ alumina activities. It may be synthesized with trivalent framework ions other than aluminum to form, for example, borosilicates, boroaluminosilicates, gallosilicates, or galloaluminosilicates structural isotypes, which are considered to constitute forms of zeolite beta. The zeolite beta may be acid extracted to form the high silica/ alumina products.

As noted previously, various aromatic conversion processes may be carried out in either or both of the liquid and vapor phases. At the relatively high temperatures involved in vapor phase reactions, it is generally accepted that water present in the feed stream is detrimental to the reaction process. While various reasons are advanced for the adverse impact of water, the most widely observed detrimental effect is probably catalyst deactivation due to dealumination. For example, U.S. Patent No. 4,197,214 to Chen et al. discloses a process for modifying various crystallined zeolite molecular sieves such as ZSM-5, ZSM-11, ZSM-12, ZSM-35, ZSM-38, faujasite, mordenite, and erionite by the inclusion of metallic ions such as zinc. Chen et al. state that high temperature steam functions by way of a hydrolysis reaction to cause loss of framework aluminum which is accompanied by the loss of the associated protons, leading to a reduction in catalytic activity. The hydrolysis reaction is said to be quite slow at temperature below 427°C (800°F). However, at higher temperatures above 482°C (900°F), the reaction rate is sufficiently fast to affect long term stability of the zeolite catalyst.

In some cases, water can be tolerated under the high temperature conditions involved in vapor phase reactions. For example, the aforementioned patent to Dwyer states that water and hydrogen sulfide are tolerable if more rapid aging of the catalyst is acceptable, but are moderately detrimental in the process. Steam stabilized zeolites are disclosed as useful in aromatic conversion processes involving alkylation such as in the production of ethylbenzene or cumene. Thus, the aforementioned patent to Ward et al. discloses that steam stabilized hydrogen Y zeolite is preferred in the alkylation of benzene to produce ethylbenzene or cumene.

The use of steam stabilized zeolites in the production of high molecular weight alkyl benzenes is disclosed in the aforementioned patent to Young in which relatively high molecular weight alkylating agents are used in either the vapor phase or the liquid phase. The zeolite catalyst may be subjected to modifying treatments involving steaming for periods ranging from one quarter to 100 hours in an atmosphere containing from 5 to 100% steam.

In hydrocarbon conversion processes involving olefin conversion, water may or may not be tolerated in the feed stream, depending on the nature of the molecular sieve employed. For example, U.S. Patent No. 4,551,438 to Miller discloses the oligomerization of olefins over molecular sieves such as ZSM-5, ZSM-11 and silicalite characterized as intermediate pore size having an effective pore aperture in the range of 5 to 6.5 angstroms. Miller discloses that the feed should contain less than 100 ppm and preferably less than 10 ppm water, as well as being low in sulfur and nitrogen. On the other hand, when employing a somewhat larger pore size molecular sieve, specifically steam stabilized zeolite Y in the conversion of $C_2$-$C_4$ olefins to motor fuels, water is described as an effective cofeed which stabilizes the catalyst and reduced the deactivation rate. As also described in U.S. Patent No. 4,740,648 to Rabo et al., co-fed water is described as a particularly desirable diluent which tends to aid in resistance of zeolite Y catalyst to coking and aging.

SUMMARY OF THE INVENTION

In accordance with the present invention there is provided an alkylation-transalkylation process involving alkylation of an aromatic substrate with a $C_2$-$C_4$ alkylating agent coupled with separation to recover a monoalkylated aromatic product and with the liquid phase transalkylation of the polyalkylated product and said aromatic substrate as defined in the claims.

BRIEF DESCRIPTION OF THE DRAWINGS

FIGURES 1a-1c and 2a-2c are graphs illustrating the results of transalkylation experiments carried out using two different zeolite Y catalysts.

FIGURES 3a-3c are graphs illustrating the results of experimental work carried out in the transalkylation of diethylbenzene using another zeolite Y catalyst.

FIGURES 4a-4c are a series of graphs showing experimental work carried out with a rare earth zeolite.

FIGURES 5a, 5b and 6 are graphs illustrating further experimental work employing a zeolite Y catalyst.

FIGURE 7 is a simplified schematic flow diagram illustrating one embodiment of the invention in which a polyethylbenzene fraction is subjected to a residue extraction step prior to transalkylation.

FIGURE 8 is a schematic illustration of a modification of the process of FIGURE 7 in which the output from the transalkylation reactor is subjected to a separation step prior to recycle.

FIGURE 9 is a simplified schematic illustration of yet another embodiment of the invention in which the bottoms fraction from an ethylbenzene column is supplied directly to a transalkylation reactor with the output of the transalkylation reactor being supplied to a downstream separation zone.

FIGURE 10 is a schematic flow diagram showing a modification of the embodiment of FIGURE 9.

FIGURE 11 is a schematic illustration of a specific embodiment for carrying out the invention employing a plurality of series connected reaction stages.

FIGURE 12 is a graph showing the results of experimental work involving dehydration of a feedstream to a transalkylation reaction carried out over a zeolite Y catalyst.

FIGURE 13 is a graph showing the results of experimental work involving dehydration of a feedstream to a transalkylation reaction carried out over another zeolite Y catalyst.


DETAILED DESCRIPTION

A preferred application of the invention involves liquid phase alkylation over a zeolite beta alkylation catalyst coupled with liquid phase transalkylation over a molecular sieve transalkylating catalyst selected from the group consisting of zeolite Y and zeolite beta. An especially preferred embodiment of the invention involves the use of zeolite beta as an alkylation catalyst and zeolite Y as a transalkylation catalyst. However, as will appear below, other molecular sieve catalysts can be employed in carrying out the present invention.

In its more general aspects, the invention involves transalkylation coupled with aromatic alkylation employing $C_2$-$C_4$ alkylating agents which, broadly stated, can be alkylating agents of the type disclosed in the aforementioned patent to Pollitzer et al., such as olefins, alkynes, alkyl halides, alcohols, ethers and esters. The most widely used alkylating agents are ethylene and propylene applied in the production of ethylbenzene and cumene, respectively. The invention is especially applicable to the ethylation of benzene under conditions in a manner in which byproduct xylene yields are reduced and the invention will be described specifically by reference to the production of ethylbenzene together with the attendant transalkylation of polyethylbenzenes.

As noted previously, a conventional process for the production of ethylbenzene involves recycling polyethylbenzenes, separated from the ethylbenzene product, to the alkylation reactor where they undergo transalkylation to yield ethylbenzene. A byproduct of this procedure is increased xylene yield in the effluent from the alkylation reactor. The presence of xylenes complicates downstream processing and separation steps. A particular impact of a significant xylene content in the product stream is that it often mandates operation of the distillation column from which the ethylbenzene is taken overhead in a manner to provide a substantial ethylbenzene content, often times 15-20% or more, in the bottom polyethylbenzene fraction. For example, ethylbenzene produced in accordance with the present invention can be employed in the production of styrene by catalytic dehydrogenation. The boiling points of ortho xylene and styrene are very close, within 1°C of one another. As a practical matter, the ethylbenzene specifications will call for a very low xylene content, normally less than 2000 ppm. In order to meet this specification, it is normally necessary to operate the ethylbenzene column under moderate distillation conditions resulting in a high ethylbenzene content in the bottoms fraction as described above. The present invention, by carrying out polyethylbenzene transalkylation in a separate reactor under relatively mild liquid phase conditions, minimizes the xylene make in the manufacturing process. This enables ethylbenzene recirculation to be reduced by limiting the ethylbenzene content in the polyethylbenzene fraction to 5 wt.% or less, and where preferred catalysts are used to further minimize xylene make, down to about 2 wt. % or less ethylbenzene.

A preferred aspect of the present invention involves supplying the polyethylbenzene fraction, including both diethylbenzene and the triethylbenzene and higher molecular weight compounds to the transalkylation reactor as contrasted with separating out a substantial portion of the diethylbenzene for recycle to the alkylation zone, as disclosed in the aforementioned patent to Wight, or separating out trialkylaromatics with transalkylation only of dialkylbenzene, as disclosed in the aforementioned patent to Barger. In this respect, depending upon the configuration of the interface of the transalkylation reactor and polyethylbenzene or other separation zones, substantially all of the diethylbenzene and substantially all or most of the triethylbenzene content will be supplied to the transalkylation reactor. In either case, the practical effect of this embodiment of the invention is that recycle to the alkylation reactor is limited to benzene and lighter components, e.g., ethylene, while most if not all of the triethylbenzenes together with diethylbenzenes are retained in the system ultimately for conversion to benzene and ethylbenzenes. This offers significant advantages over the prior art processes, not only in terms of reduced xylene makes as described previously, but also in terms of ultimate product yield.

In experimental work relative to the invention, a number of catalysts were employed in transalkylation tests carried out in an upflow, flooded-bed reactor, that is, only a liquid phase was in contact with the catalyst. The feed employed in this experimental work was an approximate 1:1 mixture of benzene and the polyethylbenzene overheads fraction from a commercial operation employing vapor phase alkylation of benzene to produce ethylbenzene. A typical feed employed in the experimental work had the composition as shown below in Table I.

Table I

| Non-Aromatic | 0.32 |
|---|---|
| Benzene | 50.241 |
| Toluene | 0.000 |
| Ethylbenzene | 6.117 |
| p+ M-Xylene | 0.000 |
| Styrene | 0.063 |
| o-Xylene | 0.066 |
| Cumene | 3.973 |
| n Propylbenzene | 7.816 |
| m + p Ethyltoluene | 2.053 |
| 1,3,5-Trimethylbenzene | 0.128 |
| o-Ethyltoluene | 0.356 |
| 1,2,4-Trimethylbenzene | 0.536 |
| 1,2,3-Trimethylbenzene | 0.401 |
| m-Diethylbenzene | 14.808 |
| o + p-Diethylbenzene | 7.328 |
| Butylbenzenes | 1.653 |
| Heavies | 4.429 |

In the experimental work, the average pressure was about 20.7 bars (300 psia) with a pressure drop across the reactor ranging from 0.34 to 1 bars (5 to 15 psi). The temperature profile across the reactor was relatively constant with an endotherm from the inlet to the outlet of less than 10°C and usually less than 5°C. The experimental runs were initiated at relatively low temperatures, usually less than 100°C and progressively increased as described later. The space velocity was maintained relatively constant at a value of 6 hr.$^{-1}$ (LHSV) based on the total hydrocarbon feed. Diethylbenzene conversions and selectivity to ethylbenzene were measured as a function of catalyst age (duration of the run) along with the production of various other components including xylenes.

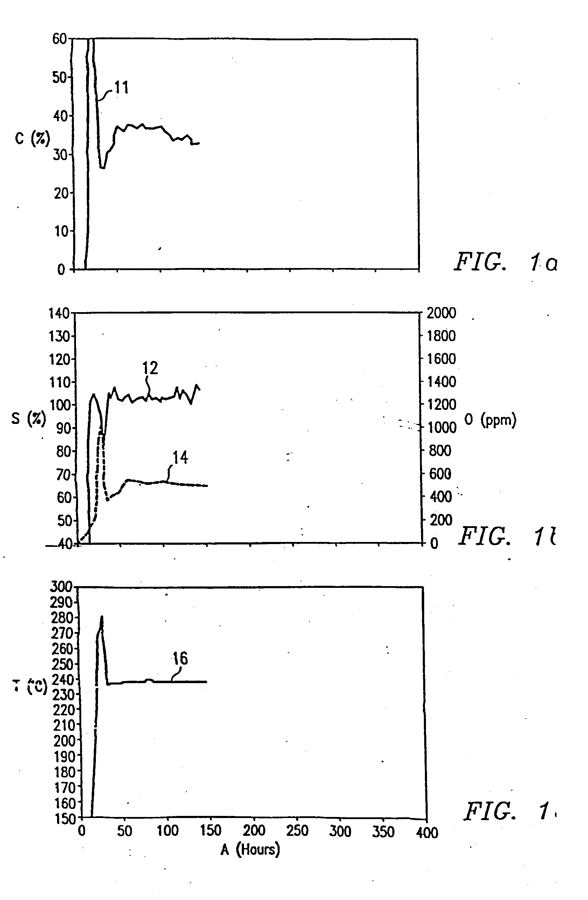

In a first test run, the catalyst used was a commercially available zeolite Y (identified herein in Catalyst A) in which the inlet temperature was progressively increased up to about 235°C and stabilized there with an average temperature increase through the reactor of only 1° or 2°C. The results of this experimental work are illustrated in FIGURES 1a-1c in which percent diethylbenzene conversion C, percent selectivity to ethylbenzene S, ortho xylene production O, in ppm, and temperature, T, °C, are plotted as curves 11, 12, 14, and 16, respectively versus the catalyst age A, in hours, on the abscissa. As can be seen from an examination of the data presented in FIGURE 1, the diethylbenzene conversion stabilized in about the 32-37% range for a reactor temperature of about 237°C with the catalyst showing very little deactivation over the duration of the run. The selectivity to ethylbenzene was virtually 100%. During the run, O-xylene production stabilized at about 400 to 500 ppm.

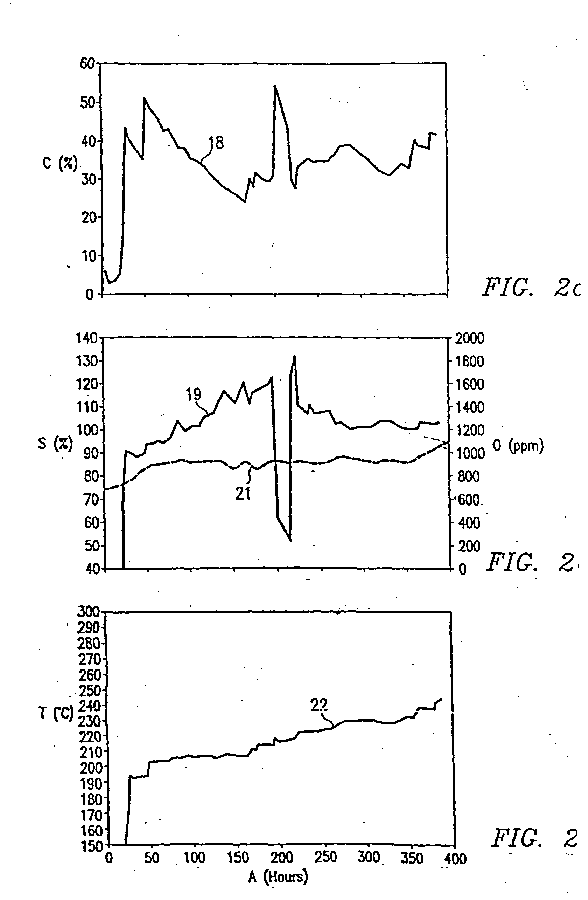

Another test run was carried out using an experimental zeolite Y identified herein as Catalyst B. The results of this run are set forth in FIGURES 2a-2c in which curves 18, 19, 21 and 22 are graphs of diethylbenzene conversion, C, selectivity to ethylbenzene, S, parts per million O-xylene, O, and temperature, T, respectively plotted as a function of catalyst Age A. In this experiment, the catalyst was run for nearly 400 hours with the temperature, after initialization, increasing slightly with time to a final value of about 240°C. As can be seen, diethylbenzene conversion was relatively good, mostly in the 30-40% range at relatively moderate temperatures. Selectivity to ethylbenzene was greater than 90% and during most of the run was virtually at 100%. The O-xylene content of the product stream stabilized at about 900 ppm.

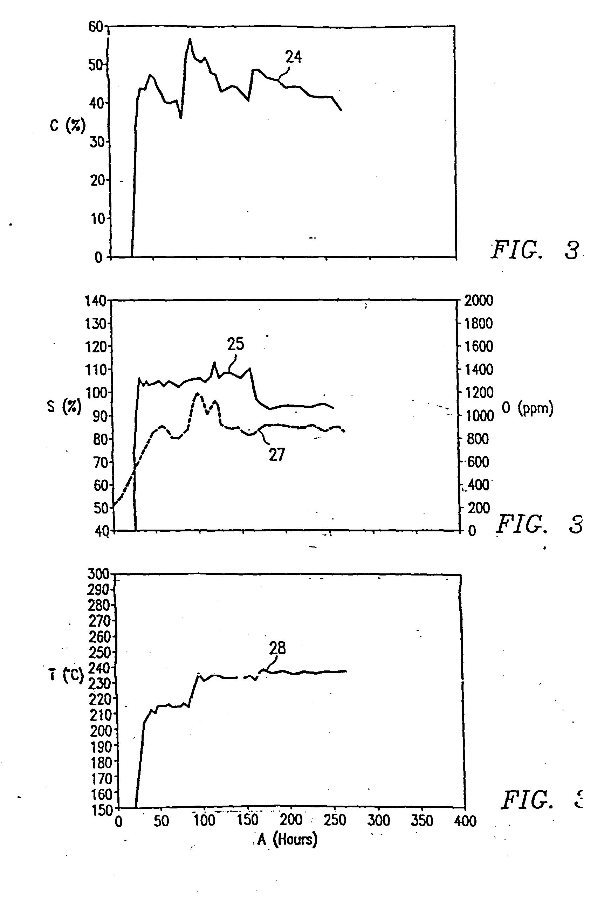

Yet another test run was carried out employing another zeolite Y catalyst identified herein as Catalyst C. The results herein in terms of diethylbenzene conversion, selectivity and as a function of time and temperature, are set forth in FIGURES 3a-3c. In FIGURE 3, curves 24, 25, 27 and 28 are graphs of diethylbenzene conversion, selectivity to ethylbenzene, O-xylene content (ppm), O, and temperature, T, °C as a function of catalyst age on the abscissa. As shown in FIGURE 3, diethylbenzene conversion was, on balance, slightly better than for catalysts A and B, and fell generally into the 40-50% range at reactor temperatures ranging from 210° to 236°C. Selectivity to ethylbenzene was more than 90% over much of the run at virtually 100%. O-xylene content stabilized at about 800-900 ppm. The catalyst showed very little deactivation over the life of the run.

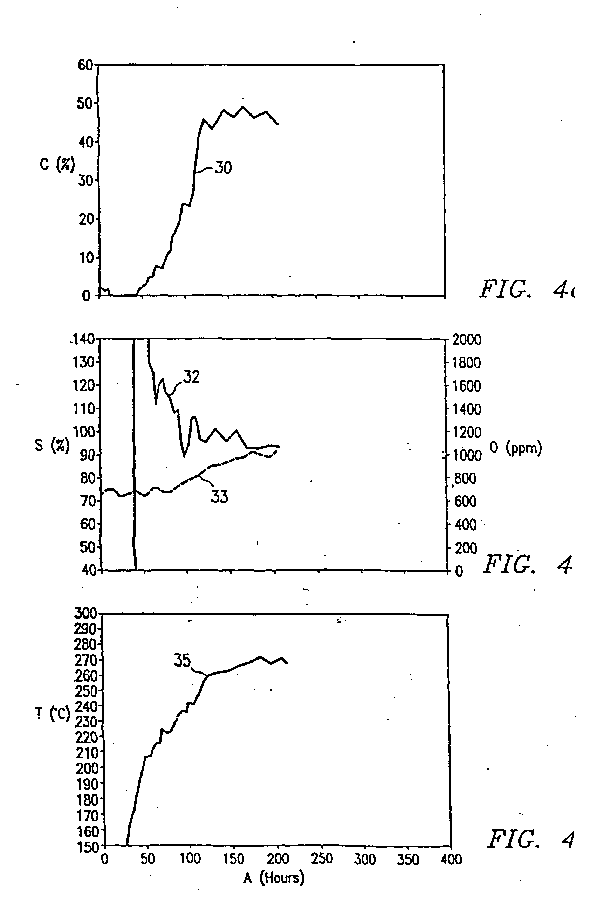

A rare earth zeolite Y identified herein as Catalyst D was employed in yet another test. The results for catalyst D are set forth in FIGURES 4a-4c with curves 30, 32, 33 and 35 representing graphs of diethylbenzene conversion, selectivity to ethylbenzene, ppm, O-xylene and temperature, respectively, as a function of catalyst age. Catalyst D showed relatively good results including diethylbenzene conversion in the 40-50% range. Initial selectivity was about 100%, with selectivity falling off slightly to about 90% toward the end of the run. While good conversion and selectivity were achieved, the reaction temperature was substantially higher than for the previous zeolite Y, rising to about 270°C at the conclusion of the run, about 210 hours.

The feeds for the experimental work depicted in FIGURES 1-4 conformed generally to the composition shown in Table I. However, the feed for the first test run (Catalyst A) was free of ortho xylene and the feed for the second run (Catalyst B) contained about 0.02% para and meta xylene.

Additional experimental work under the above-identified conditions was carried out employing three additional catalysts; Catalyst E, a cation exchange resin available from Rohm & Haas under the designation Amberlyst 15, Catalyst F, a superacidic alumina available from Harshaw-Filtrol under the designation 3998 and Catalyst G a nickel modified mordenite available from Union Carbide under the designation Ni-Cn9040. Catalyst E showed little diethylbenzene conversion and no ethylbenzene production up to the time the experiment was terminated, at about 50 hours and a temperature of 155°C, due to experimental difficulties. Catalyst F produced diethylbenzene conversions ranging from 10 to 20% at temperatures ranging from 300°-450°C with selectivity to ethylbenzene for the most part being less than 50%. Catalyst G was run for 100 hours at temperatures ranging up to 350°C and showed almost no diethylbenzene conversion. and B were also used in downflow trickle bed reactors where a substantial gas phase was present. Fresh and regenerated catalysts were used. This experimental work was carried out at pressures of about 22.8 bars (330 psig), nominal space velocities of about 10 hr$^{-1}$ (LHSV) and average reactor temperatures of about 300°C in the case of fresh catalyst A, 300°-400°C in the case of fresh Catalyst B and about 200°C in the case of the regenerated catalysts. For fresh catalyst A, initial diethylbenzene conversion was about 24% but this fell off rapidly after a few hours. The catalyst was then regenerated and under the less severe temperature conditions of about 200°C, initial diethylbenzene conversion was high, about 60% but this, again, reduced to only a few present after about 24 hours.

When employing fresh catalyst B the initial diethylbenzene conversion was over 50%, but this fell to about 20% after about 5 hours and then decreased further to only a few percent. The regenerated catalyst B, when run at the lower temperature of about 200°C, showed an initial diethylbenzene conversion of about 58% which declined to about 27% after 29 hours, at which time the run was terminated.

Yet additional experimental work was carried out employing zeolite Y identified above as catalyst B in which the feed was a relatively pure diethylbenzene mixed in approximately equal parts with benzene. Unlike the feedstock employed in the experimental work of FIGURES 1 through 4, the pure diethylbenzene feedstock contained only very small amounts of material susceptible to cracking or other conversion reactions, e.g., deethylation, to produce xylenes and was also free of xylenes. The makeup of the feedstock in this experimental work is set forth below in Table II.

Table II

| Components | Wt.% |
|---|---|
| Non-aromatics | 0.01 |
| Benzene | 56.58 |
| Toluene | 0.09 |
| Ethylbenzene | 0.01 |
| Xylenes | 0.0000 |
| n-PR-BZ | 0.02 |
| m,p-ethyltoluene | 0.03 |
| o-ethyltoluene | 0.01 |
| 124 trimethylbenzene sec-BU-BZ | 0.47 |
| 123 Trimethylbenzene m,Diethylbenzene | 27.62 |
| o,p-diethylbenzene | 14.27 |
| n-BU-BZ | 0.35 |
| Heavies | 0.54 |

In this test run, the inlet and outlet pressures were held at 21.3 (310) and 21.0 (305) bars (psig), respectively. The average temperature of the reactor was increased approximately linearly with time from an initial value of about 198° to a final value of about 298°C. The space velocity was generally held within the range of about 5.8-6.0 hr$^{-1}$ (LHSV) with the exception of about two-thirds of the way through the test where it fell to about 5.1 before recovering to the higher value.

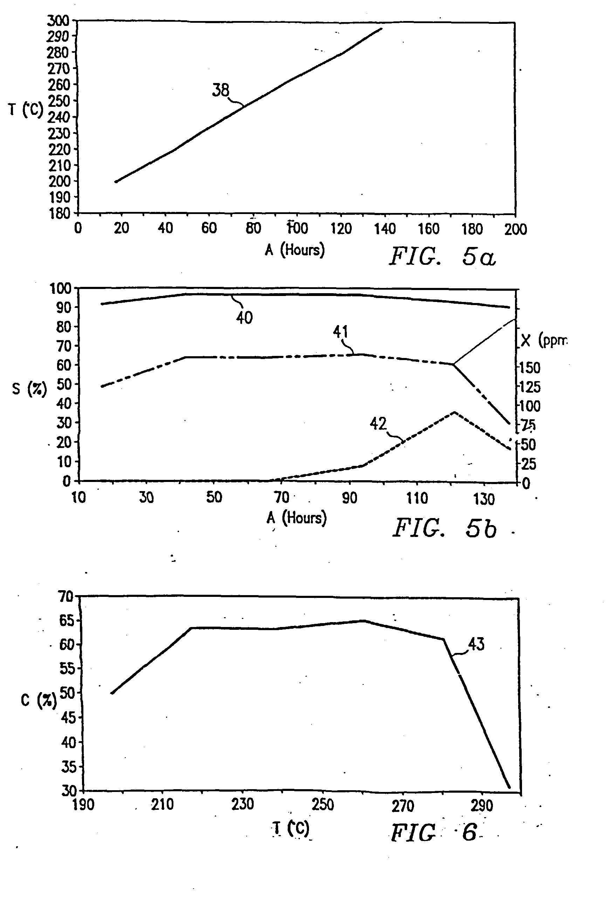

The results of this test run are set forth in FIGURES 5 and 6. In FIGURE 5a, curve 38 is a graph of temperature, T, versus catalyst A in hours on the abscissa. In FIGURE 5b curves 40 and 41 are graphs of percent selectivity to ethylbenzene and percent ethylbenzene conversion, respectively. Curve 42 is a graph of the total xylene make, X, expressed in ppm, based upon the amount of ethylbenzene produced. FIGURE 6 shows the relationship between ethylbenzene conversion and temperature. Curve 43 is a graph of ethylbenzene conversion, C, on the ordinate versus temperature, T, on the abscissa.

As indicated by the data set forth in FIGURE 5, xylene make remained low throughout the test run. No xylene was produced until the temperature was increased to about 260°C (which generally corresponds to the reduction in space velocity to about 5.1 hours$^{-1}$ as reported previously). Percent conversion remain good until the temperature was increased above 280°C. As indicated in FIGURE 6, ethylbenzene conversion appears to remain above 50% over a temperature range of 200°-290°C with the optimum range appearing to be 210° to 280°C.

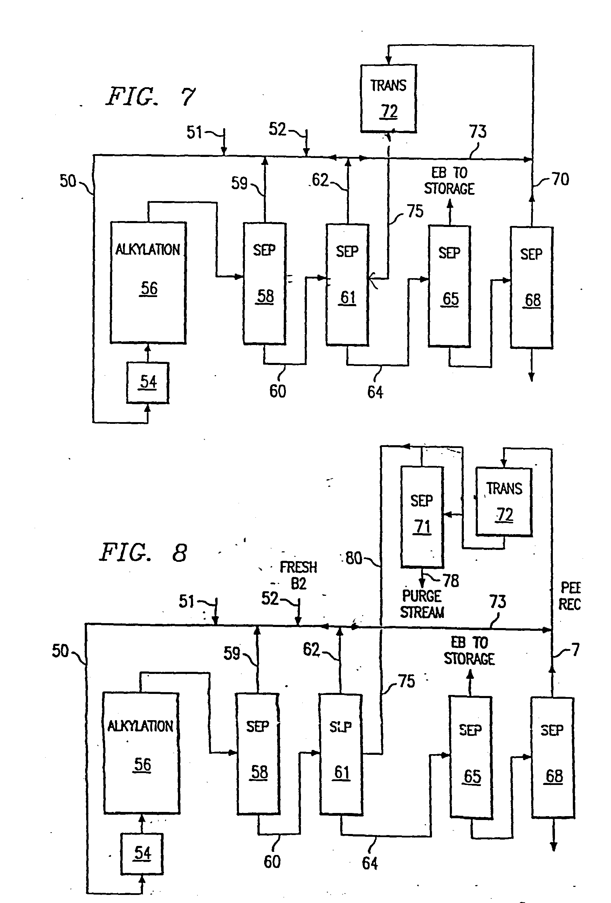

With further reference to the drawings, FIGURES 7 through 10 illustrate schematic flow diagrams illustrating different embodiments of the invention. It will be assumed for purposes of discussion that the invention is applied in the production of ethylbenzene by reaction of ethylene with benzene and that the alkylation reaction is carried out in a flooded-bed liquid phase alkylation reactor employing zeolite beta or zeolite Y as the alkylation catalyst.

Referring first to FIGURE 7, a feedstream 50 containing ethylene and benzene supplied via lines 51 and 52, respectively, is passed first to a dehydrator 54, where the water content is reduced to a level of about 100 ppm or less, preferably about 50 ppm or less, and then to an alkylation reaction zone 56. The alkylation reactor, which may comprise a plurality of series connected adiabatic reactors with interstage injection of ethylene and also interstage cooling, normally will be operated at an average temperature of about 220°C and under sufficient pressure, about 41.4 bars 600 psia) or above, to maintain the benzene in the liquid phase and at least about 2 mole percent of ethylene solubilized in the benzene. As an alternative to using adiabatic reactors, one or more isothermal reactors can be employed with suitable cooling means used to maintain a substantially constant temperature (little or no temperature differential) from the inlet to the outlet of the reactor. The effluent stream from the alkylation reactor is supplied to a prefractionation column 58 which is operated to provide a light overheads fraction including benzene which is supplied via line 59 to the alkylation reactor input and a heavier liquids fraction containing benzene, ethylbenzene separation zone 61.

The output from the prefractionation zone 58 is supplied via line 60 to abenzene separation zone 61. The overhead fraction from column 61 contains the remaining benzene which is recycled via line 62 to the alkylation reactor input. The heavier bottoms fraction from column 61 is supplied via line 64 to an ethylbenzene separation zone 65. The overheads fraction from column 65, of course, comprises ethylbenzene which is supplied to storage or to any suitable product destination. By way of example, the ethylbenzene may be used as a feedstream to a styrene plant in which styrene is produced by the dehydrogenation of ethylbenzene. The bottoms fraction containing polyethylbenzenes, heavier aromatics and preferably only a small amount of ethylbenzene, no more than 5% as discussed previously, is supplied to polyethylbenzene separation zone 68. The bottoms fraction of column 68 comprises a residue. The overhead fraction from column 68, containing polyethylbenzene, triethylbenzene (usually in relatively small quantities) and a minor amount of ethylbenzene is supplied to a transalkylation reaction zone 72. By minimizing the amount of ethylbenzene recovered from the bottom of column 65, the ethylbenzene content of the transalkylation feedstream is kept small in order to drive the transalkylation reaction in the direction of ethylbenzene production. The polyethylbenzene fraction withdrawn overhead through line 70 is mixed with benzene supplied via line 73 and then supplied to the transalkylation reactor 72. The mole ratio of benzene to polyethylbenzene should be at least 1:1 and preferably is within the range of 1:1 to 4:1. The output from the transalkylation reactor containing benzene, ethylbenzene and diminished amounts of polyethylbenzenes is supplied via line 75 to the benzene column 61.

In the process depicted in FIGURE 7, the alkylation reaction is carried out in the liquid phase with dehydration of feed to the alkylation reactor.

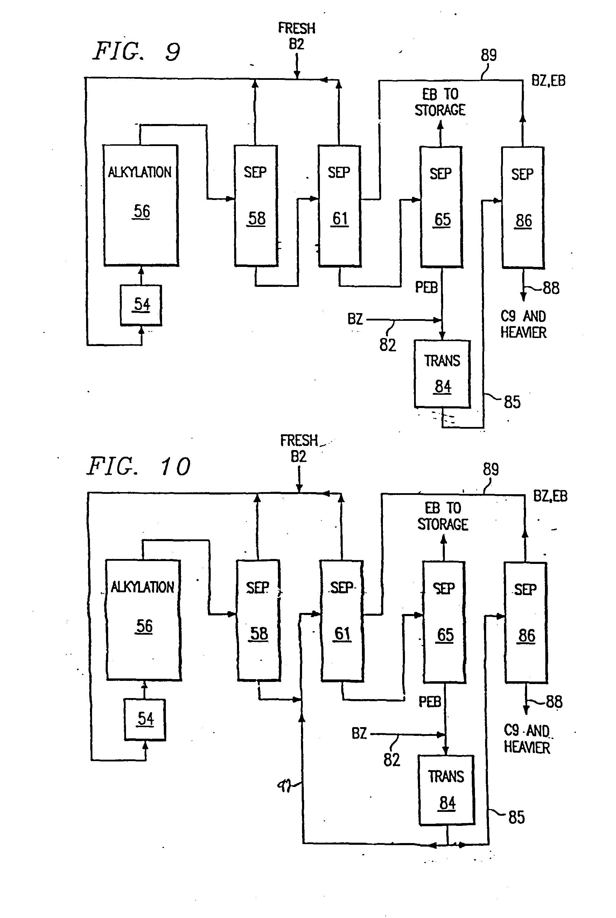

FIGURE 8 discloses a modification of the process disclosed in FIGURE 7 in which the transalkylation reactor output is subjected to further treatment prior to recycle to the separation system. The embodiment of FIGURE 8 is particularly useful in those cases in which relatively high conversion is achieved in the transalkylation reactor. In the embodiment of FIGURE 8, the alkylation reactor and separation system is identical to that of FIGURE 7 and like components are indicated by the same reference characters. However, the output from the transalkylation reactor is supplied to a secondary separation zone 77 which may take the form of a distillation column which is operated in a manner to produce a bottom purge stream withdrawn via line 78 and a recycle stream withdrawn via line 80 and supplied to the benzene column.

The purge stream containing heavy hydrocarbons is withdrawn from the system, thus providing a partially single pass system in which high molecular weight hydrocarbons are not recirculated.

FIGURE 9 illustrates yet another embodiment of the invention in which the polyethylbenzene fraction recovered from the ethylbenzene column is directly passed to a transalkylation reactor. In FIGURE 9, the same system components as shown in FIGURES 7 and 8 are designated by like reference numerals. As shown in FIGURE 9, the output from the ethylbenzene column 65 is mixed with benzene supplied via line 82 and supplied to the transalkylation reactor 84. Here, the entire polyethylbenzene fraction is subjected to transalkylation. The conditions employed in reactor 84 may be the same as described above with the ratio of benzene to polyethylbenzene ranging from 1:1 to 4:1.

It will be recognized that the procedure depicted in FIGURE 9 is similar to that of FIGURE 8 except that the entire bottoms fraction from the ethylbenzene column is subjected to the transalkylation reaction. Limiting the ethylbenzene content of the input to the transalkylation reactor to no more than 5%, preferably 2% or less is especially significant here in establishing conditions promoting the transalkylation reaction. The output from the transalkylation reactor is applied via line 85 to a post transalkylation separation zone 86 which may take the form of a distillation column operated to produce an overhead fraction that is comprised predominantly of benzene and ethylbenzene and a bottoms fraction, composed predominantly of $C_9$ and $C_{10}$ hydrocarbons such as ethyltoluene, cumene, butylbenzene, etc., which is eliminated from the recycle stream by purge line 88. The overheads fraction is recycled through line 89 to the benzene column similarly as described above.

The embodiment of FIGURE 10 is similar to that of FIGURE 10 except that the transalkylation reactor output is split, with a portion being directly supplied to the benzene column 61 via line 92 and the remainder to the separation zone 86 which is operated as described above. The configuration of FIGURE 10 provides a means for maintaining a low concentration of $C_9$ and $C_{10}$ hydrocarbons in the system and reduces the energy costs of operating column 86. Typically about 60% or more of the transalkylation reactor output is recycled directly to the benzene column 61 with the remainder being directed to the separation zone 86.

As noted previously, one of the zeolite catalysts useful as an aromatic alkylation catalyst is zeolite beta and a specific embodiment of the present invention involves the use of an aromatic alkylation catalyst comprising a modified zeolite beta, under relatively mild liquid phase alkylation conditions. The invention is especially applicable to the ethylation of benzene under mild liquid phase conditions producing little or no xylene make, and the invention will be described specifically by reference to the production of ethylbenzene. However, other alkylation reactions may be utilized in carrying out the invention. For example, the invention may be applied to the reaction of propylene with benzene to produce cumene. Also, while olefinic alkylating agents normally will be employed, other alkylating agents such as alkynes, alkyl halides, alcohols, ethers, and esters as disclosed, for example, in the aforementioned patent to Pollitzer et al., may be used. Other aromatic substrates such as toluene and xylene may also be subject to alkylation in accordance with the invention.

The activity of zeolite beta as utilized in the present invention is in direct contrast to the teachings found in the aforementioned patent to Dwyer which suggests that zeolite beta, because of its low constraint index, is unsuitable for use under the relatively severe conditions involved in the vapor phase ethylation of benzene, and in the aforementioned patent to Young, which limits its application to relatively long chain alkylating agents. Dwyer, which is directed to ethylbenzene production under relatively severe temperature conditions well above 300°C, i.e., 650°-900°F, and preferably 700°-850°F, teaches as noted above, that zeolite beta cannot be used as a catalyst in the ethylation of an aromatic substrate even under the high temperature conditions involved in the Dwyer process.

The present invention proceeds in a manner directly contrary to these prior art teachings. In the invention, zeolite beta is a highly effective catalyst for the alkylation of an aromatic substrate with low molecular weight ($C_2$-$C_4$) alkylating agents. Moreover, it is an effective alkylating agent under mild liquid phase conditions involving temperatures of 300°C or less, providing high conversion efficiency and high selectivity to monoalkylation. As noted previously, these mild reaction conditions permit the production of ethylbenzene with a xylene make which is negligible and, for all practical purposes, nonexistent.

Crystalline zeolite beta which is identified by its x-ray diffraction pattern and basic procedures for its preparation are disclosed in the aforementioned U.S. Patent No. 3,308,069 to Wadlinger et al. As described therein, zeolite beta is synthesized by the hydrothermal digestion of a reaction mixture comprising silica, alumina, an alkali or alkaline earth metal oxide or hydroxide and an organic templating agent.

The zeolite beta catalysts employed in this embodiment of the invention preferably are of ultra-low sodium content which can readily be produced by new procedures as described in detail below. Low sodium content zeolite betas are in themselves, known in the art. For example, the aforementioned patent to Wadlinger discloses zeolite beta formed by treating a dried product resulting from the digestion procedure which was exchanged continuously for 48 hours with a 2% solution of ammonium chloride. After washing free of excess chloride ions, the catalyst was dried and calcined for three hours at 538°C (1000°F) to produce an acid beta aluminosilicate having 0.07% Na content. The aforementioned patent to Calvert et al. also discloses zeolite beta of relatively low sodium content, e.g., 0.14 wt.% and 0.11 wt.% Na.

The preferred zeolite beta used in the present invention is characterized by a sodium content substantially lower than those disclosed in the patents to Wadlinger et al. and Calvert et al. It is also characterized in terms of a very high surface area, specifically at least 600 $m^2/g$ based upon the crystalline zeolite beta. The preferred zeolite beta has a low sodium content of less than 0.04 wt.% and preferably less than 0.02 wt.%, expressed as $Na_2O$. The preferred zeolite beta is produced by means of a series of ion exchange and calcination procedures carried out employing as-synthesized zeolite beta as a starting material. The synthesized zeolite beta can be produced by the hydrothermal digestion of a reaction mixture in accordance with any suitable procedure such as those disclosed in the aforementioned U.S. patents to Wadlinger et al. and Calvert et al., and the aforementioned European patent applications.

In producing modified zeolite beta as described above, the as synthesized zeolite beta is subjected to a plurality of sequential ion exchange and calcination treatments to arrive at a molecular sieve product of extremely low sodium content, substantially below the sodium content of the acid zeolite beta produced in accordance with the patent to Wadlinger as described above. In addition, the final molecular sieve product has a substantially higher surface area than that heretofore attained for zeolite beta. This embodiment of the invention involves an initial ion exchange treatment of the as-synthesized zeolite beta followed by calcination, followed by subsequent ion exchange to arrive at a product of ultra-low sodium content and high surface area.

In experimental work carried out respecting the invention, the high surface area, ultra low sodium content zeolite beta described above, was employed as a catalyst in the reaction of ethylene and benzene to produce ethylbenzene. The experimental work was carried out in an upflow reactor heated by a sand bath set at a nominal temperature of 200°C. The reactor contained 3.6 grams (7.0 ml) of catalyst in the form of 1/16" pellets based upon the zeolite-binder mixture and having a surface area of 642 $m^2/g$ as described previously. The benzene was supplied to the bottom of the reactor at a rate to provide a space velocity (LHSV) of 10 $hr^1$. Ethylene was supplied to provide a benzene/ethylene mole ratio of 5.2 with the actual mole ratio varying during the experimental work from about 5.0 to 5.3. The results of the experimental work are set forth in Table IV. The effluent analysis is set forth in weight percent for benzene and ethylbenzene and for various other components including toluene, cumene, meta-diethylbenzene, and ortho and para diethyl benzene in yields relative to ethylbenzene. The total xylene yield throughout the run was zero.

The reactor was equipped with four thermocouples spaced evenly from the inlet, TC #1, to the outlet TC #4. From the exotherm profile indicated by the thermocouples, it is evident that most of the alkylation reaction occurred in the lower portion of the catalyst bed. Thus, the effective space velocity was substantially higher than the nominal value of 10 $hr^{-1}$ (LHSV).

TABLE IV

| SAMPLE NO. 1 | 1 | 4 | 5 | 6 | 7 | 8 | 9 | 10 | 11 | 12 |
|---|---|---|---|---|---|---|---|---|---|---|
| Cat Age, Hours | 1.4 | 19. | 27.2 | 36. | 44.0 | 61. | 86. | 108. | 132. | 155. |
| PRESS PSI | 500 | 500 | 500 | 490 | 500 | 460 | 410-510 | 415-520 | 430-520 | 420-520 |
| Outlet TC 4 | 204 | 205 | 205 | 205 | 204 | 205 | 205 | 207 | 206 | 209 |
| TC 3 | 217 | 218 | 219 | 217 | 215 | 219 | 214 | 228 | 222 | 235 |
| TC 2 | 231 | 229 | 227 | 227 | 225 | 220 | 213 | 232 | 216 | 229 |
| Inlet TC 1 | 202 | 203 | 201 | 201 | 201 | 202 | 201 | 200 | 201 | 200 |
| BZ WT % | 89.90 | 75.74 | 76.22 | 76.39 | 81.44 | 87.47 | 81.11 | 75.48 | 78.60 | 75.40 |
| EB WT % | 8.754 | 20.345 | 20.031 | 19.573 | 15.632 | 9.786 | 14.850 | 19.391 | 16.735 | 19.412 |
| YIELDS RELATIVE TO EB | | | | | | | | | | |
| N-AR WT % | 0.482 | 0.367 | 0.310 | 0.347 | 0.279 | 0.357 | 0.403 | 0.391 | 0.298 | 0.436 |
| TOL PPM | 1119 | 614 | 624 | 644 | 640 | 736 | 741 | 742 | 741 | 742 |
| CUM PPM | 69 | 25 | 25 | 26 | 0 | 51 | 40 | 36 | 36 | 26 |
| M-DEB WT % | 8.971 | 10.818 | 10.574 | 10.836 | 10.459 | 15.032 | 15.286 | 14.749 | 15.728 | 14.398 |
| O+P-DEB WT % | 6.461 | 7.584 | 7.643 | 7.955 | 7.798 | 10.638 | 11.205 | 11.046 | 11.586 | 11.637 |
| TOTAL XYLENES | 0.000 | 0.000 | 0.000 | 0.000 | 0.000 | 0.000 | 0.000 | 0.000 | 0.000 | 0.000 |
| TOTAL DEB | 15.832 | 18.702 | 18.217 | 18.791 | 18.257 | 25.669 | 26.492 | 25.795 | 27.314 | 26.035 |

EP 0 485 683 B1

TABLE IV

| SAMPLE NO. 1 | 13 | 14 | 15 | 16 | 17 | 18 | 19 | 20 | 21 | 22 | 23 | 24 |
|---|---|---|---|---|---|---|---|---|---|---|---|---|
| Cat Age, Hours | 179 | 210 | 257 | 279 | 300 | 306 | 324 | 341 | 359 | 365 | 385 | 462 |
| PRESS PSI | 430-525 | 525 | 505 | 475-545 | 510 | 500 | 495 | 495 | 505 | 500 | 500 | 470 |
| Outlet TC 4 | 207 | 209 | 206 | 209 | 208 | 206 | 207 | 204 | 207 | 207 | 206 | 208 |
| TC 3 | 218 | 230 | 222 | 222 | 229 | 220 | 224 | 207 | 224 | 224 | 222 | 222 |
| TC 2 | 215 | 226 | 224 | 220 | 226 | 223 | 223 | 223 | 223 | 223 | 221 | 221 |
| Inlet TC 1 | 201 | 201 | 201 | 202 | 202 | 201 | 201 | 201 | 201 | 201 | 200 | 201 |
| BZ WT % | 80.17 | 76.76 | 78.17 | 73.54 | 83.29 | 85.21 | 74.03 | 76.09 | 69.68 | 70.01 | 72.63 | 70.88 |
| EB WT % | 15.473 | 19.000 | 17.855 | 20.942 | 11.306 | 12.114 | 20.961 | 19.206 | 24.756 | 24.353 | 22.222 | 23.708 |
| YIELDS RELATIVE TO EB | | | | | | | | | | | | |
| N-AR WT % | 0.464 | 0.348 | 0.225 | 0.240 | 0.475 | 0.430 | 0.280 | 0.275 | 0.164 | 0.270 | 0.290 | .339 |
| TOL PPM | 743 | 711 | 560 | 497 | 964 | 1032 | 573 | 541 | 545 | 538 | 558 | 599 |
| CUM PPM | 32 | 26 | 22 | 29 | 53 | 0 | 29 | 26 | 28 | 29 | 27 | 25 |
| M-DEB WT % | 15.471 | 13.246 | 9.997 | 12.593 | 23.973 | 10.045 | 11.755 | 10.784 | 10.571 | 10.860 | 10.961 | 10.088 |
| O+P-DEB WT % | 11.969 | 11.227 | 9.235 | 12.322 | 23.034 | 9.836 | 11.695 | 11.553 | 11.573 | 11.830 | 11.727 | 12.207 |
| TOTAL XYLENES | 0.000 | 0.000 | 0.000 | 0.000 | 0.000 | 0.000 | 0.000 | 0.000 | 0.000 | 0.000 | 0.000 | 0.000 |
| TOTAL DEB | 27.440 | 24.473 | 19.232 | 24.915 | 47.007 | 19.881 | 23.451 | 22.337 | 22.144 | 22.690 | 22.688 | 22.305 |

EP 0 485 683 B1

From an examination of the experimental data presented in Table IV, and bearing in mind that, for the most part, the ethylene charge to the reactor was slightly less than 20% of the stoichiometrically equivalent amount of benzene, it can be seen that the zeolite beta catalyst is highly active and shows good selectivity to ethylbenzene production. The activity of the catalyst remained substantially undiminished after the duration of the experimental work.

In carrying out this embodiment of the invention, the alkylation reaction is carried out at pressures well above the vapor pressure of the aromatic substrate at the reaction temperature involved in order to ensure that a liquid phase is retained in the reactor. In order to provide a complete liquid phase reaction, a flooded-bed format is used in which the catalyst bed is completely immersed in liquid. This can readily be accomplished using an upflow technique such as used in the experimental work and this usually will be preferred in carrying out the invention. However, downflow flooded bed operation can be accomplished by control of the outlet flow rate to ensure that the catalyst beds are covered by liquid benzene or other aromatic substrate.

Preferably, a staged reaction format is employed in order to ensure good solubility of the ethylene (or other alkylating agent) in the benzene (or other aromatic substrate) so that the entire reaction takes place in the liquid phase. In addition, use of multiple stages provides an opportunity for interstage cooling where adiabatic reactors are used or permits the use of several isothermal reaction stages.

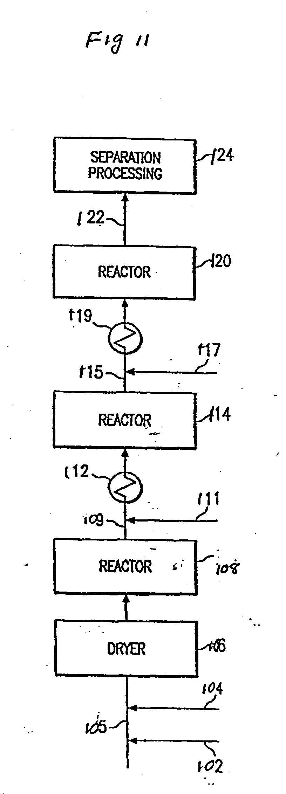

Turning now to FIGURE 11 of the drawings, there is shown a schematic illustration of a staged reactor system used for the production of ethylbenzene by the reaction of ethylene with benzene which includes a plurality of adiabatic reactors with interstage cooling and injection of ethylene. More particularly, and as illustrated in the drawing, ethylene and benzene are supplied via lines 102 and 104 to the inlet line 105 of a dehydration unit 106 which is operated in accordance with another embodiment as described hereinafter. The dehydration unit functions to dehydrate the input to the alkylation reactors so that it is essentially dry, preferably containing less than 100 ppm, more preferably, less than 50 ppm, water. By way of example, dehydrator 106 may take the form of a packed column packed with a desiccant such as silica gel or other suitable hydrophilic medium.

The dehydrator effluent is supplied to a reactor 108, the first of a plurality of series connected alkylation reactors operated in an upflow mode. Reactor 108 is operated at an average temperature of 300°C or less, and preferably, at an average temperature within the range of 200°-250°C. The pressure on reactor 108 is sufficient to maintain the benzene in the liquid phase and preferably is at least 3.45 bars (50 psi) above the vapor pressure of the benzene at the reactor temperature. Typically, the reactor pressure is within the range of 34.5-41.4 bars (500-600 psia). The remaining downstream reactors normally are operated under approximately the same conditions as the initial reactor. The effluent from the initial reactor 108 is withdrawn via line 109 and applied through a heat exchanger 112 where it is cooled. Ethylene is supplied via line 111 where it is mixed with the effluent from the first reactor 108. Preferably, the ethylene is supplied to the reactor effluent prior to cooling in order to facilitate distribution of the ethylene throughout the liquid benzene. Desirably, the cooling step is carried out to reduced the temperature of the feed mixture supplied to the second reactor 114 to a value about the same as the inlet temperature to the first reactor 108. The average temperature in the second reactor normally will be about the same as that of the first reactor. The pressure will, of necessity, be somewhat lower in order to provide for sufficient pressure gradient to accomodate flow through the system. The effluent from the second reactor 114 is supplied along with ethylene provided via line 117 to a second interstage cooling unit 119 where the charge mixture to third reactor 120 is again cooled to a temperature about equal to the inlet temperature for the first two reactors.

The output from reactor 120 is supplied via line 122 to a downstream separation and processing unit 124. In unit 124, ethylbenzene is separated and withdrawn as the product of the alkylation plant. Typically, ethylbenzene will be used as the charge to a dehydrogenation system system where it undergoes catalytic dehydrogenation in the production of styrene. Normally, benzene and ethylene will be separated in unit 124 and recycled for use in the alkylation process. Heavier polyethylbenzenes may be transalkylated with benzene to produce additional ethylbenzene as described previously.

As is conventional, a stoichiometric excess of benzene to ethylene will be supplied in the charge stock to the alkylation reactors in order to enhance selectivity for monoalkylation. Operation of the reactors to provide liquid phase alkylation under relatively mild conditions not only minimizes the xylene produced in the alkylation reaction but also enables the use of a somewhat lower benzene/ethylene mole ratio than is usually the case. Usually, the benzene/ethylene mole ratio will be 5:1 or less, and more preferably, 4:1 or less. Benzene/ethylene mole ratios as low as about 2:1 may be employed. Ratios greater than 5:1 can be used. However, there is usually little incentive to use extremely high ratios and, as a practical matter, the benzene/ethylene mole ratio will seldom exceed 15:1. The benzene/ethylation mole ratios referred to above are with respect to the overall system and for a multi-stage reaction system such as depicted in the drawing, the benzene/ethylene ratio of the feed to each stage will be less than the overall ratio.

The amount of ethylene solubilized in the benzene charge to each reactor stage will depend in part upon the number of reactor stages employed. Preferably, at least 3 reactor stages, as illustrated, will be used. Additional reactor stages may be provided, although the total number of stages normally will not exceed 8. Preferably, the pressure in each reaction stage and the amount of ethylene supplied therein is such as to provide at least 1 mole percent of ethylene solubilized in the benzene. Usually at least 2 mole percent of ethylene will be solubilized in the charge to each reactor. Preferably, unless a great many reactor stages are employed, usually the amount of ethylene solubilized in the liquid benzene phase of each reactor will be at least 4 mole percent.

The following Table V gives exemplary conditions and reaction parameters for a multistage system of the type shown in the drawing, but employing five reaction stages. As will be discussed below, Table V also illustrates a preferred mode of operation when going from one reaction stage to the next, as well as advantages accruing in the use of multiple reaction stages in the liquid phase alkylation process where the reaction pressure, while above the vapor pressure of the aromatic substrate, is below the vapor pressure of the alkylating agent at the reaction conditions.

TABLE V

| Reaction Stage | Temp C In | Temp C Out | Press (Psia) In | Press (Psia) Out | Benzene Feed Rate Moles | Benz Conv % | $C_2H_4$ feed rate Mole | $\dfrac{Bz}{C_2H_4}$ |
|---|---|---|---|---|---|---|---|---|
| 1 | 210 | 240 | 500 | 485 | 4 | 9% | .4 | 10.0 |
| 2 | 210 | 240 | 480 | 465 | 3.64 | 10% | .4 | 9.1 |
| 3 | 210 | 240 | 460 | 445 | 3.28 | 11% | .4 | 8.2 |
| 4 | 210 | 240 | 440 | 425 | 2.97 | 12% | .4 | 7.3 |
| 5 | 210 | 240 | 420 | 415 | 2.56 | 14% | .4 | 6.4 |

In Table V, the idealized reactor conditions for the ethylation of benzene with ethylene are illustrated in columns 2-5. The benzene feed rate in moles per unit time to each of the reaction stages is set forth in column 6. Benzene conversion for each reaction stage is indicated in column 7 and the ethylene feed rate in moles per unit time to each reaction zone is set forth in column 8. The last column presents the mole ratio of benzene to ethylene at the input of each of the successive reaction stages. The data presented in Table V is based upon an idealized case, assuming that benzene conversion is about 90% of theoretical based upon the feed rate of ethylene which, of course, is the limiting reactant.

At the temperature and pressure conditions depicted in Table V, the solubility of ethylene in the liquid aromatic compounds involved, including benzene, ethylbenzene and polyethylbenzene, is about 10 mole percent. The ethylene feed to the first reaction stage is controlled in order to provide an amount of ethylene near the solubility limit. Within the first reaction zone, 0.36 moles of benzene are converted and the effluent from the first reaction zone, after cooling as described previously, is applied to the second reaction zone. The aromatic feed to the second reaction zone will comprise about 3.64 moles of benzene and about 0.36 moles of ethylbenzene and polyethylbenzenes per unit time. Since the ethylated product can serve to solubilize the ethylene at the reaction conditions, the ethylene feed rate can be retained at 0.4 moles per unit time, resulting in a decreased benzene conversion rate. The relationships described above prevail when going from one reaction stage to the next, resulting in a decreased mole ratio of benzene to ethylene in each succeeding reaction stage and an increased benzene conversion rate although in the idealized case presented in Table V, the interstage injection of ethylene is maintained constant. This need not necessarily be the case. For example, the ethylene feed rate can be increased or decreased slightly from one stage to the next, or alternatively decreased and increased, so long as the overall progression across the multistage system is one of decreasing benzene/ethylene ratio with the attendant increase in benzene conversion. By way of an example of a progressively decreasing feed rate and with reference to the system depicted in Table V, the ethylene feed rate can be progressively decreased by 2-3% when going from one reaction stage to the next while retaining the characteristic of a decreasing benzene/ethylene ratio as depicted in the Table. If the ethylene feed rate is increased when going from one stage to the next, it is preferred to maintain the ethylene below the solubility limit at the temperature and pressure conditions involved in order to avoid multiphase flow through the catalyst bed.

Multistage ethylation of benzene may also be carried out in accordance with the present invention employing isothermal reaction zones. Isothermal reactors can take the form of shell and tube type heat exchangers with the alkylation catalyst deposited within the tubes and with a heat transfer medium circulated through the shell surrounding the catalyst filled tubes. The heat exchange medium will, of course, be supplied through the reactors at rates to maintain a relatively constant temperature across each reaction stage. In this case, interstage cooling will be unnecessary, although it will be preferred to inject ethylene at the front of each reaction stage.

As discussed previously, it is desirable in accordance with certain aspects of the invention to employ a dehydration step. Aromatic conversion reactions such as alkylation or transalkylation may be carried out in the vapor phase or in the liquid phase. Intermediate pore sized zeolites such as ZSM-5 (pore size of about 6 angstroms) are effective catalysts for vapor phase alkylation or transalkylation where movement of aromatic molecules in the gas phase through the molecular sieve network takes place by energy vibration. Somewhat larger pore size molecular sieves appear to be necessary to provide effective catalysts for processes such as the liquid phase alkylation of benzene. Thus, benzene, which has a kinetic diameter of about 5.9 angstroms, will enter into the molecular sieve network of an intermediate pore size molecular sieve such as ZSM-5. However, the resulting alkylated product such as ethylbenzene or cumene will not readily move through the molecular sieve channels by liquid phase displacement.

The zeolite molecular sieves employed in the present invention and having a pore size greater than 6.5 angstroms are effective catalysts under relatively mild conditions for liquid phase hydrocarbon aromatic hydrocarbon conversion reactions such as the ethylation of benzene or the transalkylation of polyethylbenzene. As noted previously, conversion takes place at relatively low temperature conditions of less than 300°C, about 275°C or less. In fact, effective ethylation or transalkylation reactions can take place in the liquid phase over larger pore size zeolite molecular sieves employed in the present invention at temperatures within the range of about 200°-250° C and such reactions can be accomplished without undesirable side reactions as may be encountered in vapor phase reaction conditions. The pressure on the reaction zone in which the conversion reaction takes place is necessarily above the vapor pressure of the aromatic substrate involved. Preferably, the reaction zone pressure is at least 3.45 bars (50 psi) above the vapor pressure. Thus, in the ethylation of benzene at 225°C to produce ethylbenzene, the reactor pressure preferably would be 24.1 bars (350 psi) or more. In general the reactor pressure may range from 17.2-68.9 bars (250-1000 psig).

While, as noted previously, water can be tolerated in vapor phase reactions, it does under the high temperature con. ditions encountered in vapor phase reaction, effect the dealumination of the catalyst with a corresponding decrease in protonated sites and a reduction in acidic catalyst activity. One would not expect a similar effect to be encountered under the relatively mild conditions of liquid phase aromatic conversion reactions and, in fact, it appears that dealumination in the presence of water does not occur under these conditions. However, by dehydrating the feed stream to the liquid phase reaction zone, . the aging quality of the catalyst is substantially increased. In fact, by decreasing the water content to well below 300 ppm, a value normally tolerated in vapor phase reactions without substantial adverse impact upon catalyst aging quality, the aging quality of the catalyst in the liquid phase condition is materially enhanced.

As noted previously, the molecular sieves employed in the present invention have pore sizes greater than 6.5 angstroms which readily accomodate movement of molecules within the molecular sieve network by a liquid phase displacement mechanism. The preferred zeolite molecular sieves, zeolites Y and beta, have a pore size within the range of 7.0-7.5 angstroms. The catalysts are not acid extracted to effect dealumination. In additional experimental work carried out relative to the invention, such larger pore size zeolite molecular sieves were employed as catalysts in the liquid phase transalkylation of diethylbenzene. Two zeolite Y catalysts were used in this experimental work. Zeolite Y is characterized by a three dimensional channel system and has an average pore size of about 7.3. Zeolite Y catalysts have silica/alumina ratios of less than 10, usually about 5-6.

In this experimental work, a mixture of benzene and a polyethylbenzene overheads fraction resulting from a vapor phase alkylation process was passed into a reactor containing a zeolite Y catalyst. The reactor was operated in a flooded, upflow mode configuration and under a pressure of about 30 psig to maintain the aromatic compounds in the liquid phase. The flow rate was sufficient to provide a space velocity (LHSV) based upon the total feed of about 3 $hr^{-1}$. The weight ratio of benzene to polyethylbenzene overheads was about 4. A typical feed composition employed in the experimental work is shown in Table VI.

Table VI

| Non-Aromatic | 0.01 |
|---|---|
| Benzene | 78.87 |
| Toluene | 0.00 |
| Ethylbenzene | 3.40 |
| p-Xylene | 0.01 |
| m-Xylene | 0.02 |
| Styrene | 0.03 |
| o-Xylene | 0.04 |
| Cumene | 1.67 |
| n Propylbenzene | 3.30 |
| m-Ethyltoluene | 0.15 |
| p-Ethyltoluene | 0.05 |
| o-Ethyltoluene | 0.04 |
| 1,3,5-Trimethylbenzene | 0.07 |
| 1,2,4-Trimethylbenzene | 0.20 |
| sec-Butylbenzene | 0.39 |
| 1,2,3-Trimethylbenzene | 0.32 |
| m-Diethylbenzene | 7.03 |
| n-Butylbenzene | 0.29 |
| p,o-Diethylbenzene | 3.90 |
| Heavies | 0.49 |

The water content of the feed was about 300 ppm. The temperature was progressively increased during the run as necessary to maintain the transalkylation reaction at 70% conversion of diethylbenzene. Over the first 11 days of the experimental run, the charge of wet feedstock was first passed into a dehydrator filled with a molecular sieve desiccant. The output from the dehydrator was passed into the reaction zone. The dried feedstock was estimated to have a water content of about 30 ppm. Thereafter, and over the remainder of the run, the wet feed was applied directly to the reactor.

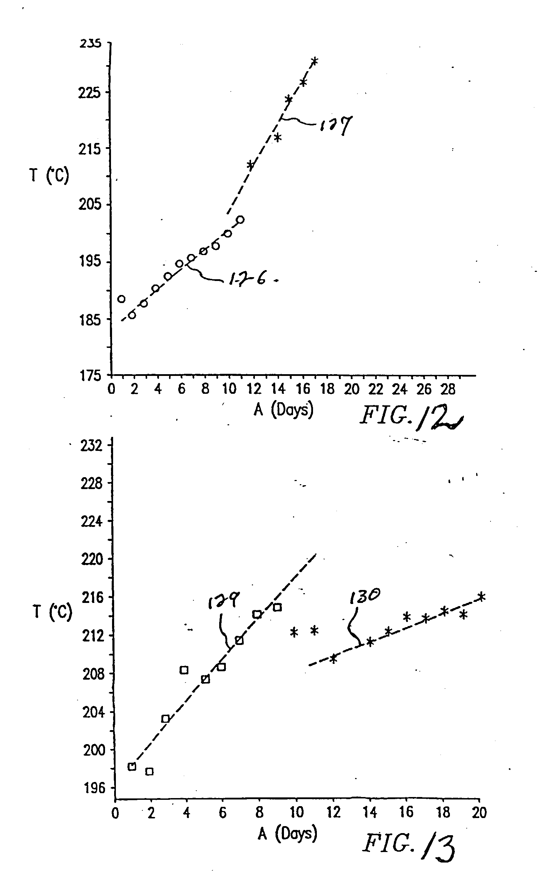

The results of the experimental work employing one of the zeolite Y catalysts are set forth in FIGURE 12. In FIGURE 12, curves 126 and 127 are graphs of temperature, T in °C necessary to maintain 70% diethylbenzene conversion plotted on the ordinant versus the age, A, of the catalyst (the duration of the run) in days plotted on the abscissa. As indicated by curve 126 for the dried feed, the catalyst exhibited an aging quality of about 1.8°C per day (average daily increase in temperature necessary to maintain 70% conversion). Curve 127 of FIGURE 12 indicates the aging quality of the catalyst when the feed stream was diverted from the dryer so that the wet feed containing about 300 ppm water was directly applied to the alkyaltion reactor. As indicated by curve 127, the aging characteristic for the catalyst more than doubled to about 3.9°C/day.

Similar experimental work was carried out using another zeolite Y catalyst in the liquid-phase transalkylation of polyethylbenzene. The feedstock employed here was the same as the feedstock used in the experimental work described immediately above. In this case, the temperature was adjusted as necessary to maintain the transalkylation reaction at 80% conversion of diethylbenzene. The space velocity was the same as employed in the previous zeolite Y experimental work, 3 hr$^{-1}$ (LHSV). The transalkylation reaction was carried out at a pressure of 300 psig in order to maintain the aromatic hydrocarbons in the liquid phase. In this test, the wet feed, containing about 300 ppm, was initially applied to the reaction vessel containing the zeolite Y. At the conclusion of nine days, the feedstream was first directed to a dehydrator containing silica gel which extracted water from the feed stream to provide a water content of about 30 ppm. The run was then continued for an additional 11 days during which dehydrated feed was supplied to the reaction zone. The results of the experimental work carried out for the second zeolite Y are illustrated in FIGURE 13, in which curves 129 and 130 are graphs of temperature T, in °C, required for 80% diethylbenzene conversion of the wet and dry feeds, respectively, plotted against catalyst age in days. As shown in FIGURE 13, the initial wet feed caused a very rapid deactivation of the catalyst. However, at the conclusion of the wet feed injection, the introduction of dry feed not only materially reduced the catalyst deactivation rate but actually enhanced the activity of the catalyst.

In addition to transalkylation, this embodiment of the invention may be employed in the liquid phase alkylation of aromatic substrates. As indicated earlier, a particularly important liquid phase alkylation reaction is the ethylation of benzene under mild liquid phase conditions which results in little or no xylene make. Other liquid phase alkylation reactions may be employed, particularly those involving the use of $C_2$-$C_4$ alkylating agents. For example, this embodiment of the invention may be employed in the reaction of propylene and benzene to produce cumene. Usually, alkylating agents will take the form of olefins. However, other alkylating agents such as alkynes, alkyl halides, alcohols, ethers and esters as disclosed, for example, in the aforementioned patent to Pollitzer, may be employed. Also, aromatic substrates other than benzene, for example, toluene or xylene, may also be subject to liquid phase alkylation in accordance with the invention.

As noted previously, dehydration of an aromatic feedstock in accordance with the invention may be carried out in conjunction with the use of a zeolite molecular sieve other than zeolite Y having a pore size within the range of 7.0-7.5 angstroms. Specifically, zeolite beta is an effective alkylation catalyst under the mild temperature conditions involved in liquid phase alkylation. The preferred zeolite beta alkylation catalysts are, as described earlier, of a very low sodium content, less than 0.04 weight percent and preferably less than 0.02 weight percent expressed as $Na_2O$, and have a high surface area, at least 600 m$^2$/g. The zeolite beta has a silica/alumina ratio of about 20-25.

In the alkylation of benzene, both the benzene feedstock and the ethylene (or other alkylating agent) may contain water. Accordingly, it will be preferred to pass both the benzene and the ethylene through a dehydration unit. While separate dehydrators may be used for the two feed components, usually the ethylene and benzene will be mixed in the mixed feed stream and applied to the dehydration unit and from there to the liquid phase reactor.

In the application of this aspect of the invention to the transalkylation of polyalkylbenzenes, all or part of the feed to the transalkylation reactor may be subject to a prior dehydration step. Normally, the transalkylation of polyalkyl benzenes will be carried out in conjunction with a prior alkylation step with the output from the alkylation reactor being subjected to one or more separation steps resulting in a polyalkylbenzene component which is combined with benzene and then passed to the transalkyaltion reaction zone operated under liquid phase disproportionation conditions as discussed previously.

Where the invention involves a transalkylation process carried out in conjunction with a liquid phase alkylation step proceeded by a dehydration step as described above, the polyalkylbenzene component supplied to the transalkylation reactor should be substantially free of water and it normally will be necessary to subject only the benzene component to a dehydration step. However, in other applications of the invention, it may be necessary to subject the polyalkylbenzene component to a dehydration step prior to its introduction to the transalkylation reactor. For example, the transalkylation procedure may be carried out in combination with a vapor phase alkylation procedure which tolerates water in the feed stream or in which water is additionally added, for example, as disclosed in the aforementioned patent to Barger et al. In this case, it may be necessary to subject both the polyethylbenzene component and the benzene component to dehydration prior to passage to the transalkylation reactor.

**Claims**

1.    An alkylation-transalkylation process for the preparation of alkylated aromatics, the steps comprising:

   a) supplying a feedstock containing an aromatic substrate (Fig.7.52) into a reaction zone containing a molecular sieve aromatic alkylation catalyst (Fig.7-56);
   b) supplying a $C_2$-$C_4$ alkylating agent (Fig.7-51) to said reaction zone;
   c) operating said reaction zone at temperature and pressure conditions to maintain said aromatic substrate in the liquid phase and causing alkylation of said aromatic substrate by said alkylating agent in the presence of said catalyst to produce an alkylated product comprising a mixture of mono-alkylated and polyalkylated aromatic products;

d) recovering said alkylated product from said reaction zone and supplying said product from said reaction zone to a first separation zone for the separation of said aromatic substrate (Fig. 7-61);

e) operating said separation zone to produce a lower boiling fraction comprising said aromatic substrate and a higher boiling fraction comprising a mixture of monoalkylated aromatic-polyalkylated aromatic mixture;

f) supplying said higher boiling fraction from said separation zone to a second separation zone (Fig.7-65);

g) operating said second separation zone to produce a second lower boiling fraction comprising monoalkylated aromatic product and a higher boiling fraction comprising heavier polyalkylated aromatic product;

h) supplying at least a portion of said polyalkylated aromatic product including substantially all of the dialkylated and trialkylated aromatics in said polyalkylated product to a transalkylation reaction zone containing a molecular sieve transalkylation catalyst (Fig. 7-72);

i) supplying said aromatic substrate (Fig. 7-73) to said transalkylation zone;

j) operating said transalkylation reaction zone under temperature and pressure conditions to maintain said aromatic substrate in the liquid phase and effective to cause disproportionation of said polyalkylated aromatic fraction to arrive at a disproportionation product having a reduced polyalkylated aromatic content and an enhanced monoalkylated aromatic content;

k) supplying at least a portion of said disproportionation product (Fig. 7-75) to said first recited separation zone (Fig. 7-61).

2. The process of Claim 1, wherein said aromatic substrate comprises benzene and said alkylating agent is an ethylating or propylating agent wherein the polyalkylated aromatic content of said disproportionation product includes dialkyl and trialkyl benzenes.

3. The process of Claim 2, further comprising prior to step k) in claim 1, supplying the output from said transalkylation zone to a third separation zone and operating said third separation zone to produce a lower boiling fraction comprising a benzene-monoalkyl benzene component and a higher boiling fraction comprising a polyalkyl benzene component and supplying said benzene-monoalkyl component to said first recited separation zone in accordance with step k) of Claim 1.

4. The method of Claim 3, wherein said alkylating agent is an olefin.

5. The method of Claim 4, wherein said alkylating agent is ethylene.

6. The process of Claim 1, wherein said aromatic substrate comprises a benzene feedstock and said alkylation catalyst comprises a molecular sieve selected from the group consisting of zeolite beta and zeolite Y and said transalkylation catalyst comprises a molecular sieve selected from the group consisting of zeolite Y and zeolite beta.

7. The method of Claim 6, wherein said alkylation catalyst comprises zeolite beta.

8. The method of Claim 7, wherein said alkylating agent is ethylene.

9. The method of Claim 8, wherein said transalkylation catalyst is zeolite beta or zeolite Y.

10. The process of Claim 1, wherein the feedstock of step a) is benzene, and the transalkylation reaction zone of step j) is operated at an average temperature at least 50°C below the average temperature of the alkylation reaction zone.

11. The method of Claim 10, wherein said alkylating agent is ethylene and said alkylation reaction zone is operated under temperature and pressure conditions to cause vapor phase ethylation of said benzene and said transalkylation reaction zone is operated at an average temperature at least 100°C below the average temperature of said alkylation reaction zone.

12. The method of Claim 11 wherein said second separation zone is operated to produce a said second lower boiling fraction comprising ethylbenzene and said second higher boiling fraction comprising a heavy polyethylbenzene fraction containing no more than 5 wt.% ethylbenzene and wherein the aromatic substrate supplied to said transalkylation zone is benzene and polyalkylated aromatic product applied to said transalkylation reaction zone comprises said heavy polyethylbenzene fraction.

**13.** The method of Claim 12, further comprising the step of supplying said disproportionation product to a separation zone, operating said zone to produce a lower boiling fraction comprising benzene and ethylbenzene and a higher boiling polyethylbenzene fraction and supplying said lower boiling fraction to said benzene separation zone in accordance with step i).

**14.** The method of Claim 12, wherein said transalkylation catalyst comprises a molecular sieve selected from the group consisting of zeolite Y and zeolite beta.

**15.** The method of Claim 14, wherein said transalkylation zone is operated at a space velocity (LHSV) based upon benzene and alkyl benzenes which is less than the space velocity (LHSV) in said primary reaction zone based upon benzene.

**16.** The method of Claim 15, wherein said transalkylation zone space velocity is less than one half the space velocity of said primary alkylation zone.

**Patentansprüche**

**1.** Alkylierungs-Transalkylierungsverfahren für die Herstellung von alkylierten Aromaten, die Stufen umfassend:

a) Liefern eines Einsatzmaterials, enthaltend ein aromatisches Substrat (Fig. 7-52), in eine Reaktionszone, enthaltend einen Molekülsiebaromatenalkylierungskatalysator (Fig. 7-56),
b) Liefern eines $C_2$-$C_4$ Alkylierungsmittels (Fig. 7-51) in die Reaktionszone,
c) Betreiben der Reaktionszone bei Temperatur- und Druckbedingungen unter Beibehalten des aromatischen Substrats in der flüssigen Phase und Erzeugen von Alkylierung des aromatischen Substrats durch das Alkylierungsmittel in der Anwesenheit des Katalysators unter Herstellen eines alkylierten Produkts, umfassend eine Mischung von monoalkylierten und polyalkylierten aromatischen Produkten,
d) Gewinnen des alkylierten Produkts aus der Reaktionszone und Liefern des Produkts aus der Reaktionszone zu einer ersten Trennzone für die Trennung des aromatischen Substrats (Fig. 7-61),
e) Betreiben der Trennzone unter Herstellen einer niedriger siedenden Fraktion, umfassend das aromatische Substrat, und einer höher siedenden Fraktion, umfassend eine Mischung aus monoalkylierter Aromaten-polyalkylierter Aromaten Mischung,
f) Liefern der höher siedenden Fraktion aus der Trennzone zu einer zweiten Trennzone (Fig. 7-65),
g) Betreiben der zweiten Trennzone unter Herstellen einer zweiten niedriger siedenden Fraktion, umfassend monoalkyliertes aromatisches Produkt, und einer höher siedenden Fraktion, umfassend schwereres polyalkyliertes aromatisches Produkt,
h) Liefern mindestens eines Teils des polyalkylierten aromatischen Produkts, einschließlich im wesentlichen aller dialkylierten und trialkylierten Aromaten in dem polyalkylierten Produkt, zu einer Transalkylierungsreaktionszone, enthaltend einen Molekülsiebtransalkylierungskatalysator (Fig. 7-72),
i) Liefern des aromatischen Substrats (Fig. 7-73) zu der Transalkylierungszone,
j) Betreiben der Transalkylierungsreaktionszone unter Temperatur- und Druckbedingungen unter Beibehalten des aromatischen Substrats in der flüssigen Phase und wirksam, Disproportionierung der polyalkylierten aromatischen Fraktion zu erzeugen, um bei einem Disproportionierungsprodukt mit einem verminderten polyalkylierten Aromaten-Gehalt und einem erhöhten monoalkylierten Aromaten-Gehalt zu gelangen,
k) Liefern mindestens eines Teils des Disproportionierungsprodukts (Fig. 7-75) zu der ersten aufgezählten Trennzone (Fig. 7-61).

**2.** Verfahren nach Anspruch 1, wobei das aromatische Substrat Benzol umfaßt, und das Alkylierungsmittel ist ein Ethylierungs- oder Propylierungsmittel, wobei der polyalkylierte Aromaten-Gehalt des Disproportionierungsprodukts Dialkyl- und Trialkylbenzole einschließt.

**3.** Verfahren nach Anspruch 2, ferner umfassend vor Stufe k) in Anspruch 1, Liefern des Auslasses aus der Transalkylierungszone zu einer dritten Trennzone und Betreiben der dritten Trennzone unter Herstellen einer niedriger siedenden Fraktion, umfassend eine Benzol-Monoalkylbenzolkomponente, und einer höher siedenden Fraktion, umfassend eine Polyalkylbenzol-Komponente, und Liefern der Benzol-Monoalkyl-Komponente zu der ersten aufgezählten Trennzone in Übereinstimmung mit Stufe k) von Anspruch 1.

**4.** Verfahren nach Anspruch 3, wobei das Alkylierungsmittel ein Olefin ist.

**5.** Verfahren nach Anspruch 4, wobei das Alkylierungsmittel Ethylen ist.

**6.** Verfahren nach Anspruch 1, wobei das aromatische Substrat ein Benzoleinsatzmaterial umfaßt, und der Alkylierungskatalysator umfaßt ein Molekülsieb, ausgewählt aus der Gruppe, bestehend aus Zeolith beta und Zeolith Y, und der Transalkylierungskatalysator umfaßt ein Molekülsieb, ausgewählt aus der Gruppe, bestehend aus Zeolith Y und Zeolith beta.

**7.** Verfahren nach Anspruch 6, wobei der Alkylierungskatalysator Zeolith beta umfaßt.

**8.** Verfahren nach Anspruch 7, wobei das Alkylierungsmittel Ethylen ist.

**9.** Verfahren nach Anspruch 8, wobei der Transalkylierungskatalysator Zeolith beta oder Zeolith Y ist.

**10.** Verfahren nach Anspruch 1, wobei das Einsatzmaterial von Stufe a) Benzol ist, und die Transalkylierungsreaktionszone von Stufe j) wird bei einer Durchschnittstemperatur von mindestens 50°C unterhalb der Durchschnittstemperatur der Alkylierungsreaktionszone betrieben.

**11.** Verfahren nach Anspruch 10, wobei das Alkylierungsmittel Ethylen ist, und die Alkylierungsreaktionszone wird unter Temperatur- und Druckbedingungen betrieben, Dampfphasenethylierung des Benzols zu erzeugen, und die Transalkylierungsreaktionszone wird bei einer Durchschnittstemperatur von mindestens 100°C unterhalb der Durchschnittstemperatur der Alkylierungsreaktionszone betrieben.

**12.** Verfahren nach Anspruch 11, wobei die zweite Trennzone betrieben wird, die zweite niedriger siedende Fraktion, umfassend Ethylbenzol, und die zweite höher siedende Fraktion, umfassend eine schwere Polyethylbenzolfraktion, enthaltend nicht mehr als 5 Gew.% Ethylbenzol, zu erzeugen, und wobei das zu der Transalkylierungszone gelieferte aromatische Substrat Benzol ist, und polyalkyliertes aromatisches Produkt, der Transalkylierungsreaktionszone zugeführt, umfaßt die schwere Polyethylbenzolfraktion.

**13.** Verfahren nach Anspruch 12, ferner umfassend die Stufe des Lieferns des Disproportionierungsprodukts zu einer Trennzone, Betreiben der Zone unter Erzeugen einer niedriger siedenden Fraktion, umfassend Benzol und Ethylbenzol, und einer höher siedenden Polyethylbenzolfraktion, und Liefern der niedriger siedenden Fraktion zu der Benzoltrennzone in Übereinstimmung mit Stufe i).

**14.** Verfahren nach Anspruch 12, wobei der Transalkylierungskatalysator ein Molekülsieb, ausgewählt aus der Gruppe, bestehend aus Zeolith Y und Zeolith beta, umfaßt.

**15.** Verfahren nach Anspruch 14, wobei die Transalkylierungszone mit einer Raumgeschwindigkeit (LHSV), basierend auf Benzol und Alkylbenzolen, betrieben wird, welche geringer als die Raumgeschwindigkeit (LHSV) in der primären Reaktionszone, basierend auf Benzol, ist.

**16.** Verfahren nach Anspruch 15, wobei die Transalkylierungszonenraumgeschwindigkeit geringer als eine Hälfte der Raumgeschwindigkeit der primären Alkylierungszone ist.

**Revendications**

**1.** Un procédé d'alkylation-transalkylation pour la préparation d'aromatiques alkylés, les étapes comprenant:

    a) fournir une charge d'alimentation contenant un substrat aromatique (fig. 7-52) dans une zone de réaction contenant un tamis moléculaire de catalyseur d'alkylation aromatique (fig. 7-56);
    b) fournir un agent d'alkylation $C_2$-$C_4$ (fig. 7-51) à cette zone de réaction;
    c) faire fonctionner cette zone de réaction à des conditions de température et pression pour maintenir ce substrat aromatique dans la phase liquide et provoquer l'alkylation de ce substrat aromatique par cet agent d'alkylation en présence de ce catalyseur pour produire un produit alkylé comprenant un mélange de produits aromatiques monoalkylés et polyalkylés;
    d) récupérer ce produit alkylé depuis cette zone de réaction et amener ce produit depuis cette zone de réaction vers une première zone de séparation pour la séparation de ce substrat aromatique (fig. 7-61);
    e) faire fonctionner cette zone de séparation pour produire une fraction à point d'ébullition inférieur comprenant ce substrat aromatique et une fraction à un point d'ébullition plus élevé comprenant un mélange d'aromatiques monoalkylés-mélange d'aromatiques polyalkylés;

f) amener cette fraction à point d'ébullition plus élevé depuis cette zone de séparation vers une seconde zone de séparation (fig. 7-65);

g) faire fonctionner cette seconde zone de séparation pour produire une seconde fraction à un point d'ébullition plus faible comprenant un produit aromatique monoalkylé et une fraction à point d'ébullition plus élevé comprenant un produit aromatique polyalkylé plus lourd;

h) amener au moins une partie de ce produit aromatique polyalkylé comprenant sensiblement la totalité des aromatiques dialkylés et trialkylés dans ce produit polyalkylé vers une zone de réaction de transalkylation contenant un tamis moléculaire de catalyseur de transalkylation (fig.7-72);

i) amener ce substrat aromatique (fig.7-73) vers cette zone de transalkylation;

j) faire fonctionner cette zone de réaction de transalkylation sous des conditions de température et de pression pour maintenir ce substrat aromatique dans la phase liquide et efficace pour provoquer un disproportionnement de cette fraction aromatique polyalkylée pour parvenir à un produit de disproportionnement ayant une teneur en produits aromatiques polyalkylés réduite et une teneur en produits aromatiques monoalkylés accrue;

k) fournir au moins une partie de ce produit de disproportionnement (fig. 7-75) vers cette première zone de séparation citée (fig. 7-61).

2. Procédé selon la revendication 1, dans lequel ce substrat aromatique comprend du benzène et cet agent d'alkylation est un agent d'éthylation ou de propylation où la teneur en produits aromatiques polyalkylés de ce produit de disproportionnement comprend des dialkylbenzènes et trialkylbenzènes.

3. Procédé de la revendication 2, comprenant en outre avant l'étape k) de la revendication 1 l'amenée du produit de sortie de cette zone de transalkylation vers une troisième zone de séparation et faire fonctionner cette troisième zone de séparation pour produire une fraction à point d'ébullition plus faible comprenant un composant de benzène-monoalkylbenzène et une fraction à point d'ébullition plus élevée comprenant un composant de polyalkylbenzène et amener ce composant de benzène-monoalkylbenzène vers cette première zone de séparation mentionnée en accord avec l'étape k) de la revendication 1.

4. Procédé de la revendication 3, dans lequel l'agent d'alkylation est une oléfine.

5. Procédé de la revendication 4, dans lequel l'agent d'alkylation est l'éthylène.

6. Procédé de la revendication 1, selon lequel ce substrat aromatique comprend une charge d'alimentation de benzène et ce catalyseur d'alkylation comprend un tamis moléculaire choisi dans le groupe consistant en zéolite béta et zéolite Y et ce catalyseur de transalkylation comprend un tamis moléculaire choisi dans le groupe consistant en zéolite Y et zéolite béta.

7. Procédé de la revendication 6, selon lequel le catalyseur d'alkylation comprend un zéolite béta.

8. Procédé selon la revendication 7 dans lequel cet agent d'alkylation est l'éthylène.

9. Procédé de la revendication 8 selon lequel le catalyseur de transalkylation est la zéolite béta ou zéolite Y.

10. Procédé selon la revendication 1, dans lequel la charge d'alimentation de l'étape a) est le benzène, et la zone de réaction de transalkylation de l'étape j) fonctionne à une température moyenne d'au moins 50°C inférieure à la température moyenne de la zone de réaction d'alkylation.

11. Procédé de la revendication 10, selon lequel cet agent d'alkylation est l'éthylène et cette zone de réaction d'alkylation fonctionne à des conditions de température de pression de façon à provoquer une éthylation en phase vapeur de ce benzène et cette zone de réaction de transalkylation fonctionne à une température moyenne au moins 100°C inférieure à la température moyenne de cette zone de réaction d'alkylation.

12. Procédé de la revendication 11 dans lequel cette seconde zone de séparation fonctionne de façon à produire cette seconde fraction à point d'ébullition plus faible comprenant l'éthylbenzène et cette seconde fraction à point d'ébullition plus élevé comprenant une fraction de polyéthylbenzène lourde ne contenant pas plus de 5% en poids d'éthylbenzène et dans lequel le substrat aromatique amené dans cette zone de transalkylation est le benzène et le produit aromatique polyalkylé appliqué à cette zone de réaction de transalkylation comprend cette fraction de polyéthylbenzène lourde.

13. Procédé selon la revendication 12 comprenant en outre l'étape d'amener ce produit de disproportionnement vers une zone de séparation, faire fonctionner cette zone de façon à produire une fraction à point d'ébullition plus faible comprenant le benzène et l'éthylbenzène et une fraction de polyéthylbenzène à point d'ébullition plus élevé et amener cette fraction à point d'ébullition plus faible vers cette zone de séparation du benzène en accord avec l'étape i).

14. Procédé selon la revendication 12 au cours duquel ce catalyseur de transalkylation comprend un tamis moléculaire choisi dans le groupe consistant en zéolite Y et en zéolite béta.

15. Procédé selon la revendication 14 dans lequel cette zone de transalkylation fonctionne à une vitesse spatiale (LHSV) basée sur le benzène et les alkyl benzènes qui est inférieure à la vitesse spatiale (LHSV) dans cette zone de réaction principale basée sur le benzène.

16. Procédé selon la revendication 15 au cours duquel cette vitesse spatiale dans la zone de transalkylation est inférieure à la moitié de la vitesse spatiale dans cette zone d'alkylation principale.

FIG. 1a

FIG. 1l

FIG. 1

FIG. 2c

FIG. 2

FIG. 2

*FIG. 3*

*FIG. 3*

*FIG. 3*

FIG. 4(

FIG. 4

FIG. 4

FIG. 5a

FIG. 5b

FIG. 6

FIG. 7

FIG. 8

*FIG. 9*

*FIG. 10*

Fig 11

SEPARATION PROCESSING /124

122

REACTOR /120

119

115    117

REACTOR /114

112

111

109

REACTOR /108

DRYER /106

104

105

102

*FIG. 12*

*FIG. 13*